# FASCICULE DE BREVET EUROPEEN

(11) **EP 1 321 130 B1**
(45) Date de publication et mention de la délivrance du brevet: **30.11.2005**
(21) Numéro de dépôt: 02293142.2
(22) Date de dépôt: 18.12.2002
(51) Int. Cl.: A61K 7/06, A61K 7/11

(54) **Composition de coiffage repositionnable comprenant des copolymères (méth)acryliques**
Haarzusammensetzung enthaltend (Meth)acrylatcopolymere für die umformbare Haarformung
Reshapable hair styling composition comprising (meth)acrylic copolymers

(30) Priorité: 20.12.2001 US 23330
(43) Date de publication de la demande: 25.06.2003
(73) Titulaire: L'OREAL, 75008 Paris (FR)
(72) Inventeur: Rollat-Corvol, Isabelle, 75017 Paris (FR); Samain, Henri, 91570 Bievres (FR); Perron, Béatrice, 78350 Jouy en Josas (FR); Restle, Serge, 95390 Saint-Prix (FR)
(74) Mandataire: Le Blainvaux Bellegarde, Françoise

(56) Documents cités:
- EP-A- 0 761 199
- EP-A- 0 985 401
- EP-A- 1 174 113
- WO-A-00/57846
- WO-A-02/09656
- WO-A-99/08652
- FR-A- 2 760 360
- US-A- 4 196 190
- US-A- 5 501 851
- US-A- 5 658 558
- US-A- 6 149 898
- US-B1- 6 214 328
- US-B1- 6 294 158

## Description

La présente invention concerne une composition de coiffage repositionnable qui est une composition rincée.

La fixation de la coiffure est un élément important du coiffage, et implique le maintien d'une forme qui a déjà été réalisée. Conformément à l'invention, le terme « composition de coiffage » concerne tout type de composition capillaire pouvant être utilisé pour effectuer le coiffage.

Les compositions de coiffage les plus courantes sur le marché des produits cosmétiques pour la mise en forme et/ou le maintien de la coiffure sont des compositions de spray comprenant une solution, habituellement à base d'alcool ou d'eau, et un ou plusieurs composés, généralement des résines polymères. L'une des fonctions de résines polymères est de former des liens entre les cheveux. Ces matières, également appelées fixateurs, se trouvent souvent en mélange avec différents adjuvants cosmétiques. Cette solution est généralement conditionnée soit dans un récipient aérosol approprié, qui est pressurisé à l'aide d'un propulseur, soit dans un flacon pompe.

De nombreuses compositions de coiffage existant à ce jour présentent le même inconvénient : elles ne sont pas destinées à permettre à la coiffure d'être modifiée ultérieurement en une forme recherchée, qui est autre que celle initialement formée, sans recommencer de nouveau les opérations de coiffage et de fixation. Par ailleurs, sous différents types de stress, la coiffure a tendance à prendre une forme permanente indésirable qui ne peut pas être facilement modifiée. De plus, au cours du procédé de coiffage, on peut rechercher les effets bénéfiques du conditionnement des cheveux, tels qu'une facilité de peignage et l'aspect doux au toucher des cheveux.

Les documents brevet US-B1-6 214 328, EP-A-0 985 401, US-A-5 658 558, US-A-4 196 190 et US-A-5 501 851 décrivent des compositions de coiffage comprenant des polymères de type BA/HEMA/MMA.

Un objet de l'invention est une composition de coiffage repositionnable selon la revendication 1.

Un autre objet de l'invention est une composition de coiffage rincée repositionnable comprenant, dans un véhicule cosmétiquement acceptable, (1) au moins un copolymère selon la revendication 1, et (2) au moins un tensioactif. La composition fournit un effet repositionnable et est de préférence une composition rincée dont, par exemple, des shampoings et des conditionneurs.

Un autre objet de l'invention est une composition de coiffage rincée repositionnable comprenant, dans un véhicule cosmétiquement acceptable, (1) au moins un copolymère selon la revendication 1, et (2) au moins un agent conditionneur.

La composition fournit un effet repositionnable et est de préférence un shampoing ou un conditionneur.

Un autre objet de l'invention est une composition de coiffage repositionnable comprenant, dans un véhicule cosmétiquement acceptable, (1) au moins un copolymère (méth)acrylique comprenant : (a) d'environ 30 à environ 40 % en poids de motifs issus d'au moins un monomère choisi parmi les monomères d'acrylate de n-butyle, (b) d'environ 2 à environ 10 % en poids de motifs issus d'au moins un monomère choisi parmi les monomères de méthacrylate de 2-hydroxyéthyle, et (c) d'environ 50 à environ 70 % en poids de motifs issus d'au moins un monomère choisi parmi les monomères d'acrylate de 2-éthylhexyle.

Les pourcentages en poids des motifs (a), (b) et (c) sont basés sur le poids total de chaque type de monomère utilisé par rapport au poids total de tous les monomères utilisés.

Un autre objet de l'invention est une composition de coiffage repositionnable comprenant au moins un copolymère (méth)acrylique, comme décrit ci-dessus, caractérisée en ce que ladite composition de coiffage repositionnable est sous forme de spray, d'aérosol, de mousse, de gel, de lotion, de crème, de dispersion ou d'émulsion.

Un autre objet de l'invention est un procédé de traitement cosmétique des cheveux, comprenant l'application aux cheveux avant la mise en forme d'une coiffure desdits cheveux d'une composition comprenant au moins un copolymère (méth)acrylique, comme décrit ci-dessus, ladite composition étant rincée.

Un autre objet de l'invention est un procédé de déformation des cheveux, comprenant : (1) l'application sur les cheveux avant la mise en forme initiale de la coiffure d'une composition comprenant au moins un copolymère (méth)acrylique, comme décrit ci-dessus, ladite composition étant rincée, et (2) ensuite la mise en forme de la coiffure au moins une fois, et en ce qu'on n'a pas besoin de composition ou de chaleur supplémentaire.

Le terme « (méth)acrylate » englobe aussi bien le terme acrylate que le terme méthacrylate. De même, le terme « acide (méth)acrylique » englobe aussi bien l'acide acrylique que l'acide méthacrylique. On entend par « agent de réticulation polyfonctionnel » tel qu'utilisé ici un agent de réticulation ayant une fonctionnalité moyenne supérieure à 1, par exemple supérieure à 1,8, et de plus par exemple d'environ 2,0 ou plus. Mais la fonctionnalité moyenne est inférieure à environ 6, par exemple inférieure à environ 4, et de plus par exemple d'environ 3 ou moins. On entend par « composition rincée » toute composition qui est formulée pour être rincée après application sur les cheveux.

La composition rincée peut être sous toutes les formes classiques de compositions cosmétiques rincées y compris, mais sans s'y limiter, les shampoings, conditionneurs, lotions de rinçage des cheveux, compositions de permanente, compositions d'ondulation, compositions de teinture des cheveux, produits à utiliser avant ou après un traitement de teinture des cheveux, produits à utiliser avant ou après un traitement de permanente, compositions de défrisage, produits à utiliser avant ou après un traitement de défrisage, et des associations de ceux-ci. Un shampoing présente un effet nettoyant sur les cheveux et peut également présenter un effet conditionneur. Un conditionneur présente un effet conditionneur sur les cheveux sans un effet nettoyant.

On entend par composition de coiffage « repositionnable» une composition de coiffage donnant un coiffage qui peut être restauré ou modifié sans appliquer à nouveau une matière ou de la chaleur. Par exemple, pour restaurer ou modifier la coiffure dans le cas « d'alourdissement » ou de perte de mise en pli (décoiffage), on a besoin d'aucune nouvelle matière telle que l'eau ou une forme quelconque d'agent de fixation, ni de chaleur supplémentaire. Ainsi, par fournir un effet « repositionnable » on entend fournir une coiffure qui peut être restaurée ou modifiée sans appliquer de nouvelle matière ou une chaleur supplémentaire. L'efficacité de la composition peut être durable, par exemple de 10-24 heures, conduisant à un effet coiffant durable. D'autres termes pouvant être synonymes de repositionnable incluent repositionnable, redéformable, remoulable, recoiffable, ré-arrangeable et remodelable.

Dans un mode de mise en oeuvre de l'invention, le copolymère (méth)acrylique de telles compositions de coiffage repositionnables peut être sous forme d'émulsion ou de dispersion. Toutes les émulsions comprennent une phase continue et au moins une phase dispersée. On entend généralement par « dispersion » un système multiphasique où au moins une phase contient des particules discrètes distribuées dans une phase externe continue liquide, solide ou gazeuse. Une partie du polymère peut exister sous forme de particule discrète dans une phase aqueuse. Les dispersions sont possibles grâce à l'utilisation de certains composants qui sont insolubles dans le système aqueux. Par « dispersion » on entend également que ce n'est pas nécessairement l'ensemble du polymère qui doit être insoluble dans l'eau ; une partie du polymère peut être soluble dans le mélange aqueux. Il peut être souhaitable qu'une dispersion reste stable dans des conditions ambiantes. Dans un mode de mise en oeuvre, les dispersions sont stables à température ambiante pendant plus d'environ 30 jours, par exemple pendant plus d'environ 90 jours, pendant plus d'environ 180 jours, et pendant plus d'environ 360 jours. Une dispersion est jugée stable dès lors que les particules discrètes de la phase interne restent distribuées dans l'ensemble de la phase continue (phase externe).

Dans un mode de mise en oeuvre, de telles dispersions peuvent être mélangées avec d'autres dispersions ou d'autres additifs connus tels que des charges, des plastifiants, des pigments (tels que le noir de carbone), des sols de silice et d'autres agents de nivellement, agents mouillants, agents anti-mousse, et stabilisants connus.

Les monomères cités dans (a) constituent de 10 à 50 % en poids de la quantité totale de monomères utilisés. Dans un mode de mise en oeuvre, ils peuvent constituer de 15 à 50 % en poids de la quantité totale de monomères utilisés.
Les monomères cités dans (a) sont les monomères de (méth)acrylates de butyle tels que par exemple les (méth)acrylates de n-butyle, les (méth)acrylates de t-butyle, les (méth)acrylates d'isobutyle, les (méth)acrylates de 2-décylbutyle et les (méth)acrylates de 2-méthylbutyle. Dans un mode de mise en oeuvre, les monomères cités dans (a) sont choisis parmi l'acrylate de n-butyle.

Les monomères cités dans (b) constituent de 2 à 50 % en poids de la quantité totale de monomères utilisés. Dans un mode de mise en oeuvre, ils peuvent constituer de 2 à 25 % en poids de la quantité totale de monomères utilisés. Dans un mode de mise en oeuvre distinct, ils peuvent constituer de 10 à 50 % en poids de la quantité totale de monomères utilisés, par exemple de 10 à 30 % en poids de la quantité totale de monomères utilisés. Les monomères cités dans (b) sont les monomères de (méth)acrylate d'hydroxyalkyle tels que par exemple les (méth)acrylates de 2-hydroxyéthyle, les (méth)acrylates d'hydroxypropyle, les (méth)acrylates de 2,3-dihydroxypropyle, les (méth)acrylates de 4-hydroxybutyle, et les (méth)acrylates de 2-hydroxypropyle. Dans un mode de mise en oeuvre, les monomères cités dans (b) sont choisis parmi les monomères acrylate de 2-hydroxyéthyle, méthacrylate de 2-hydroxyéthyle et acrylate d'hydroxypropyle et plus particulièrement le méthacrylate de 2-hydroxyéthyle.

Les monomères cités dans (c) constituent de 30 à 80 % en poids de la quantité totale de monomères utilisés et de préférence de 50 à 70 % en poids. Dans un mode de mise en oeuvre distinct, ils peuvent constituer jusqu'à 50 % en poids de la quantité totale de monomères utilisés. Le monomère copolymérisable éventuel cité dans (c) peut être choisi parmi (i) les monomères de (méth)acrylate d'alkyle, de préférence les (méth)acrylates d'alkyle en C₁-C₂₄ tels que revendiqués, (ii) les monomères polaires et (iii) les monomères à insaturation éthylénique polymérisables par voie radicalaire libre. Les monomères de (méth)acrylate d'alkyle sont les (méth)acrylates d'isobornyle, les (méth)acrylates d'iso-octyle, les (méth)acrylates de 2-éthylhexyle et des mélanges de ceux-ci. Dans un autre mode de mise en oeuvre, les motifs issus des monomères de (méth)acrylate de 2-éthylhexyle constituent de 30 à 50 % en poids de la quantité totale de monomères utilisés.
Les monomères polaires utiles incluent l'acide (méth)acrylique, l'acide itaconique, la N-vinylpyrrolidone, le N-vinylcaprolactame, les (méth)acrylamides substitués (tels que les N,N-diméthyl(méth)acrylamides et le N-octyl(méth)acrylamide), le (méth)acrylate de diméthylaminoéthyle, le (méth)acrylonitrile, le (méth)acrylate de 2-carboxyéthyle, l'anhydride maléique, et des mélanges de ceux-ci. Un autre exemple de monomères polaires utiles est le monoacrylate de méthoxypolyéthylèneglycol 550 disponible auprès de Sartomer Co. sous le nom commercial CD553. Les monomères utiles à insaturation éthylénique polymérisables par voie radicalaire libre incluent le styrène et des esters de vinyle en C₁-C₄ tels que l'acétate de vinyle, le propionate de vinyle, et des mélanges de ceux-ci.

On peut inclure un ou plusieurs agents de réticulation polyfonctionnels. Des exemples d'agents de réticulation incluent, mais sans s'y limiter, ceux choisis parmi le divinylbenzène, les diacrylates d'alkyle (tels que ceux choisis parmi le diacrylate de 1,2-éthylèneglycol, le diacrylate de 1,4-butanediol, le diacrylate de 1,6-hexanediol, le diacrylate de 1,8-octanediol et le diacrylate de 1,12-dodécanediol), les triacrylates d'alkyle et les tétra-acrylates d'alkyle (tels que le triacrylate de triméthylolpropane et le tétra-acrylate de pentaérythritol), les cétones aromatiques à insaturation monoéthylénique (telles que la 4-acryloxybenzophénone), les aziridine-amides multifonctionnels (tels que la 1,1'-(1,3-phénylènedicarbonyl)bis[2-méthylaziridine], la 2,2,4-triméthyladipoylbis[2-éthylaziridine], la 1,1'-azélaoylbis[2-méthylaziridine] et la 2,4,6-tris(2-éthyl-1-aziridinyl)-1,3,5-triazine), les réticulants à ions métalliques (tels que le cuivre, le zinc, le zirconium et le chrome) et des mélanges de ceux-ci. Dans un mode de mise en oeuvre, les réticulants à ions métalliques sont choisis parmi les esters chélatés d'acide ortho-titanique vendus sous la désignation commerciale TYZOR et disponibles sur le marché auprès de E.I. du Pont de Nemours Co. Dans un autre mode de mise en oeuvre, le TYZOR est le TYZOR AA, qui est l'acétylacétonate de titane. Dans encore un autre mode de mise en oeuvre, l'agent de réticulation est le diacrylate de 1,6-hexanediol.

Les agents de réticulation, lorsqu'ils sont utilisés, peuvent représenter jusqu'à environ 10 parties en poids, typiquement d'environ 0,1 à environ 2 parties en poids du mélange copolymérisable total sur la base de 100 parties en poids des monomères cités dans (a), (b) et (c), lorsqu'ils sont présents.

Dans un mode de mise en oeuvre, le copolymère peut être éventuellement obtenu avec un ou plusieurs amorceurs hydrosolubles et/ou oléo-solubles, qui sont utiles dans la préparation des émulsions (méth)acryliques. De tels amorceurs, par exposition à la chaleur, génèrent des radicaux libres qui amorcent la (co)polymérisation des monomères de (méth)acrylate de butyle, des monomères de (méth)acrylate d'hydroxyalkyle et les composants éventuels de comonomère et d'agent de réticulation. Dans un mode de mise en oeuvre, on utilise un ou plusieurs amorceurs hydrosolubles. Les amorceurs hydrosolubles convenables incluent, mais sans s'y limiter, ceux choisis parmi le persulfate de potassium, le persulfate d'ammonium, le persulfate de sodium, et des mélanges de ceux-ci ; les amorceurs oxydo-réducteurs tels que le produit de réaction des persulfates cités ci-dessus et des agents réducteurs tels que ceux choisis parmi le métabisulfite de sodium et le bisulfite de sodium; et l'acide 4,4'-azobis(4-cyanopentanoïque) et ses sels solubles (par ex. de sodium, de potassium). Dans un autre mode de mise en oeuvre, l'amorceur hydrosoluble est le persulfate de potassium.

Les amorceurs oléo-solubles convenables incluent, mais sans s'y limiter, ceux choisis parmi les composés azoïques tels que VAZO 64 (le 2,2'-azobis(isobutyronitrile) et VAZO 52 (le 2,2'-azobis(2,4-diméthylpentanenitrile)), tous deux disponibles auprès de E.I. du Pont de Numours Co. ; et les peroxydes tels que le peroxyde de benzoyle, le peroxyde de lauroyle et des mélanges de ceux-ci. Dans un mode de mise en oeuvre, l'amorceur thermique oléo-soluble est le 2,2'-azobis(isobutyronitrile). Lorsqu'il(s) est (sont) utilisé(s), l'(les) amorceur(s) peut (peuvent) représenter d'environ 0,05 à environ 1 partie en poids, également d'environ 0,1 à environ 0,5 partie en poids sur la base de 100 parties en poids du mélange copolymérisable total.

Dans un autre mode de mise en oeuvre, le copolymère peut être éventuellement obtenu avec un ou plusieurs agents de transfert de chaîne. Des exemples d'agents de transfert de chaîne utiles incluent, mais sans s'y limiter, ceux choisis parmi le tétrabromure de carbone, les alcools, les mercaptans, et des mélanges de ceux-ci. Dans un mode de mise en oeuvre, l'agent de transfert de chaîne est choisi parmi l'iso-octylthioglycolate et le tétrabromure de carbone. Le mélange copolymérisable peut en outre comprendre jusqu'à environ 0,5 partie en poids d'un ou plusieurs agents de transfert de chaîne, typiquement d'environ 0,01 % en poids à environ 0,5 partie en poids, s'ils sont utilisés, également d'environ 0,05 partie en poids à environ 0,2 partie en poids, sur la base de 100 parties en poids du mélange copolymérisable total.

La polymérisation par l'intermédiaire des techniques d'émulsion peut nécessiter la présence d'un ou plusieurs émulsifiants (que l'on peut également appeler agents émulsifiants ou tensioactifs). Les émulsifiants utiles pour la présente invention incluent ceux choisis parmi les tensioactifs anioniques, les tensioactifs non ioniques, et des mélanges de ceux-ci.

Les tensioactifs anioniques utiles comme émulsifiants incluent, mais sans s'y limiter, ceux dont la structure moléculaire inclut au moins une moitié hydrophobe choisie parmi les alkyles en environ C₆ à environ C₁₂, les alkylaryles en environ C₆ à environ C₁₂, et les alcényles en environ C₆ à environ C₁₂ et au moins un groupement anionique choisi parmi les sulfates, les sulfonates, les phosphates, les polyoxyéthylènesulfates, les polyoxyéthylènesulfonates, les polyoxyéthylènephosphates, etc., et les sels de tels groupements. Dans un mode de mise en oeuvre, lesdits sels sont choisis parmi les sels de métaux alcalins, les sels d'ammonium, les sels d'amino tertiaire, etc. Des exemples commerciaux représentatifs des tensioactifs anioniques utiles comme émulsifiants incluent les laurylsulfates de sodium, disponibles auprès de Stepan Chemical Co. sous forme de POLYSTEP B-3 ; les lauryléthersulfates de sodium, disponibles auprès de Stepan Chemical Co. sous forme de POLYSTEP B-12 ; les dodécylbenzènesulfonates de sodium, disponibles auprès de Rhodia Chimie sous forme de SIPONATE DS-10; et les alkylènepolyalcoxyammoniumsulfates, disponibles auprès de PPG Industries sous forme de MAZON SAM-211.

Les tensioactifs non ioniques utiles comme émulsifiants incluent, maissans s'y limiter, ceux dont la structure moléculaire comprend un produit de condensation d'une moitié aliphatique organique et/ou alkyle aromatique avec un oxyde d'alkylène hydrophile tel que l'oxyde d'éthylène. L'HLB (balance lipophile-hydrophile) de tensioactifs non ioniques utiles comme émulsifiants est d'environ 10 ou supérieure, par exemple d'environ 10 à environ 20. L'HLB d'un tensioactif est une expression de la balance de la taille et de la résistance des groupements hydrophiles (hydro-attracteurs ou polaires) et des groupements lipophiles (oléo-attracteurs ou non polaires) du tensioactif. Des exemples commerciaux des tensioactifs non ioniques utiles dans la présente invention incluent, mais sans s'y limiter, les nonylphénoxy ou octylphénoxy poly(éthylèneoxy)éthanols disponibles auprès de Rhodia Chimie sous forme respectivement de la série IGEPAL CA ou CO ; les éthoxylates d'alcools secondaires en C₁₁-C₁₅ disponibles auprès d'Union Carbide sous forme de la série TERGITOL 15-S ; et les esters d'acides gras du sorbitane oxyéthylénés disponibles auprès d'ICI Chemicals sous forme de la série de tensioactifs TWEEN.

Dans un mode de mise en oeuvre, une polymérisation en émulsion de cette invention est réalisée en présence d'un ou plusieurs tensioactifs anioniques comme émulsifiants. Une gamme utile de concentration d'émulsifiant est d'environ 0,5 à environ 8 % en poids, par exemple d'environ 1 à environ 5 % en poids, sur la base du poids total de tous les monomères.

Dans un mode de mise en oeuvre, les copolymères (méth)acryliques sont des émulsions ou des dispersions de copolymères (méth)acryliques. Les émulsions et les dispersions de copolymères (méth)acryliques peuvent être préparées par un procédé semi-continu de polymérisation en émulsion. Dans le procédé, une fiole est chargée d'un mélange de monomères amorce comprenant de l'eau désionisée (Dl), du tensioactif, des monomères de (méth)acrylate de butyle cités dans (a), des monomères de (méth)acrylate d'hydroxyalkyle cités dans (b), et les composants éventuels tels que les monomères copolymérisables cités dans (c), des agents de réticulation polyfonctionnels, des agents de transfert de chaîne, des modificateurs de pH et d'autres additifs. Le mélange est agité et chauffé sous une atmosphère inerte telle qu'une couverture d'azote. Lorsque le mélange a atteint la température d'induction, typiquement d'environ 50°C à environ 70°C, le premier amorceur est ajouté pour amorcer la polymérisation et on laisse la réaction se dérouler de façon exothermique. Lorsque la réaction d'amorçage est terminée, la température du lot (batch) est ensuite augmentée jusqu'à la température de réaction d'alimentation, d'environ 70°C à environ 85°C. A la température de réaction d'alimentation, la pré-émulsion de monomères comprenant de l'eau Dl, du tensioactif, des monomères de (méth)acrylate de butyle cités dans (a), des monomères de (méth)acrylate d'hydroxyalkyle cités dans (b), et des composants éventuels tels que les monomères copolymérisables cités dans (c), des agents de réticulation polyfonctionnels, des agents de transfert de chaîne, et d'autres additifs est ajoutée à la fiole agitée sur une période de temps, typiquement de 2 à 4 heures, pendant que la température est maintenue. A la fin de la réaction d'alimentation, la deuxième charge d'amorceur, si on l'utilise, est ajoutée à la réaction pour réduire encore les monomères résiduels dans l'émulsion/la dispersion. Après une heure supplémentaire de chauffage, le mélange est refroidi jusqu'à température ambiante (environ 23°C) et l'émulsion/la dispersion est récupérée pour une évaluation.

Dans un mode de mise en oeuvre, le pH de l'émulsion/la dispersion préparée à l'aide de ce procédé est d'environ 2 à environ 3. L'acidité de l'émulsion/la dispersion peut être modifiée suivant la formation de l'émulsion/la dispersion à l'aide d'un ou plusieurs modificateurs de pH tels que des solutions basiques (par ex. des solutions d'hydroxyde de sodium, d'hydroxyde d'ammonium, etc.) ou des solutions tampons (par ex. du bicarbonate de sodium, etc.) à des taux d'acidité inférieurs. Dans un autre mode de mise en oeuvre, le pH est de 7 ou inférieur. Dans encore un autre mode de mise en oeuvre, le pH est dans la gamme de 2 à 6.

Dans un mode de mise en oeuvre de l'invention, les copolymères (méth)acryliques peuvent être neutralisés dans l'émulsion/la dispersion et/ou dans la composition par un ou plusieurs agents neutralisants. Les agents neutralisants convenables peuvent être choisis parmi des bases organiques, minérales ou organo-minérales, telles que les aminométhylpropanols, les hydroxydes de sodium et de potassium, les amines primaires, secondaires et tertiaires, les ammoniacs, les dérivés de ceux-ci, et les associations de ceux-ci.

Dans un mode de mise en oeuvre, les émulsions (méth)acryliques de l'invention peuvent également contenir un ou plusieurs additifs classiques, tels que des plastifiants, des colorants, des charges, des anti-oxydants, et des stabilisants des U.V. De tels additifs peuvent être utilisés s'ils n'altèrent pas les propriétés repositionnables de la composition.

Dans encore un autre mode de mise en oeuvre de l'invention, le copolymère (méth)acrylique a une température de transition vitreuse (Tg) allant d'environ - 100°C à environ 15°C. Selon la présente invention, la Tg du copolymère (méth)acrylique est obtenue à la suite de l'application du copolymère (méth)acrylique dans un milieu aqueux ou hydro-alcoolique sur une matrice, puis du séchage jusqu'à poids constant. La température de transition vitreuse est déterminée par la méthode d'analyse thermique différentielle (DSC).

Dans un mode de mise en oeuvre de l'invention, le copolymère (méth)acrylique est choisi parmi les copolymères (méth)acryliques non ioniques et faiblement anioniques. Faiblement anionique signifie que le taux de motifs issus de monomères anioniques dans le copolymère est inférieur à environ 5 % en poids.

Dans un mode de mise en oeuvre de l'invention, le copolymère (méth)acrylique est présent en une quantité allant de 0,01 à 15 % en poids, du poids total de la composition pour donner un effet repositionnable.

La composition peut en outre comprendre tout véhicule cosmétiquement acceptable qui ne gêne pas substantiellement les propriétés adhésives du ou des copolymères (méth)acryliques selon l'invention. Le choix de véhicule est adapté au procédé d'application choisi. Dans un mode de mise en oeuvre, le véhicule cosmétiquement acceptable peut être choisi parmi l'eau, les solvants miscibles à l'eau tels que les alcools inférieurs, par ex. les alcools aliphatiques à chaîne ramifiée et droite en C₁ à C₄, et des associations de ceux-ci. Dans un mode de mise en oeuvre, le copolymère (méth)acrylique est insoluble dans le véhicule cosmétiquement acceptable.

Le véhicule peut également comprendre un ou plusieurs solvants supplémentaires. Par exemple, on peut utiliser d'autres solvants s'évaporant rapidement, tels que l'hexaméthyldisiloxane (HMDS) ; les silicones cycliques (D₄ et D₅) ; les alcanes en C₄ - C₁₀ dont les isoparaffines telles que Permethyl 97A et Isopar C ; l'acétone ; les hydrofluoroéthers (HFE), etc.

La composition selon l'invention peut en outre comprendre au moins un constituant connu en cosmétique qui ne gêne pas substantiellement les propriétés repositionnables d'au moins un copolymère (méth)acrylique. De tels constituants peuvent être choisis parmi les, mais ne sont pas limités aux: agents réducteurs (tels que les thiols); silanes (tels que l'aminopropyltriéthoxysilane) ; matières grasses ; épaississants ; plastifiants ; agents anti-mousse ; agents hydratants ; charges ; filtres solaires (tels que filtres UV) ; agents actifs de soin des cheveux ; parfums ; conservateurs ; tensioactifs cationiques, anioniques, non ioniques, et amphotères (tels que zwitterioniques) ; polymères cationiques, anioniques, non ioniques, et amphotères (tels que zwitterioniques) autres que les polymères de l'invention ; polyols ; protéines ; provitamines ; vitamines ; colorants ; produits de teinture; produits de décoloration ; et agents de modification du pH. Les compositions peuvent également contenir un agent conditionneur tel que par exemple les silicones, les esters gras, les alcools gras, les hydrocarbures à longue chaîne, les émollients, les lubrifiants, les polymères, les tensioactifs, les composés de lanoline, les céramides, les protéines, les hydrylosats de protéine, et d'autres dérivés protéiques. On entend par « agent conditionneur » tel qu'utilisé ici tout agent dont la fonction est d'améliorer les propriétés cosmétiques des cheveux, par exemple la douceur, la facilité de démêlage, le toucher, et l'absence d'électricité statique. Dans un mode de mise en oeuvre, ledit au moins un agent conditionneur est choisi parmi les tensioactifs cationiques, les polymères cationiques, et les silicones. Les agents conditionneurs représente de 0,001 % à 20 % en poids, par exemple de 0,01 % à 10 % en poids, encore par exemple de 0,1 % à 3 % en poids, par rapport au poids total de la composition.

Dans un mode de mise en oeuvre, ledit constituant additionnel est choisi parmi les polymères tels que les polymères anioniques, cationiques, amphotères (tels que zwitterioniques), et non ioniques, de préférence les polymères cationiques et des associations de ceux-ci. On entend par « polymère » tel qu'utilisé ici les homopolymères et les copolymères, les copolymères étant issus de plus d'un type de monomère, par exemple de deux, trois, quatre types de monomères différents ou plus.

Les polymères cationiques comprennent des groupements cationiques ou des groupements pouvant être transformées en groupements cationiques. Des exemples convenables de polymères cationiques, qui peuvent être utilisés selon la présente invention, sont ceux pouvant être choisis parmi les polymères comprenant au moins un groupement choisi parmi les groupements amines primaires, les groupements amines secondaires, les groupements amines tertiaires, et les groupements amines quaternaires, dans lesquels ledit au moins un groupement fait partie de la chaîne polymère ou est directement relié à celle-ci, ayant un poids moléculaire moyen en poids allant d'environ 500 à environ 5 000 000, par exemple d'environ 1000 à environ 3 000 000.

Parmi ces polymères, on peut citer plus particulièrement les polymères conditionneurs cationiques suivants :
(1) les homopolymères et copolymères issus de monomères choisis parmi les esters (méth)acryliques et les amides (méth)acryliques comprenant des motifs d'au moins une des formules suivantes : 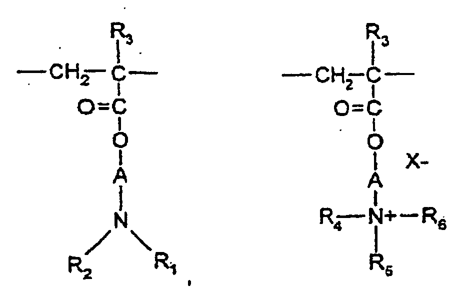 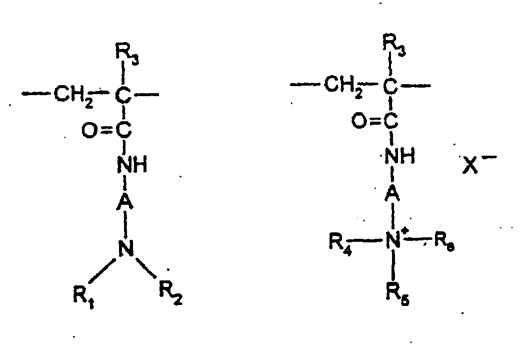 dans lesquelles chaque R₃ est choisi indépendamment parmi l'hydrogène et des groupements CH₃ ; chaque A est choisi indépendamment parmi des groupements alkyle linéaires et ramifiés comprenant de 1 à 6 atomes de carbone et des groupements hydroxyalkyle comprenant de 1 à 4 atomes de carbone ; chaque R₄, R₅ et R₆ est choisi indépendamment parmi les groupements alkyle comprenant de 1 à 18 atomes de carbone et les groupements benzyle ; chaque R₁ et R₂ est choisi indépendamment parmi l'hydrogène et les groupements alkyle comprenant de 1 à 6 atomes de carbone ; et que chaque X⁻ est choisi indépendamment parmi les anions de sulfate de méthyle et les anions d'halogénure, tels que les anions chlorure ou bromure.
   Dans un mode de mise en oeuvre, les copolymères de la famille (1) comprennent en outre au moins un motif issu de monomères choisis parmi les (méth)acrylamides, les diacétones (méth)acrylamides, les (méth)acrylamides substitués sur l'azote par un groupement choisi parmi les alkyle inférieurs, les acides (méth)acryliques, les esters d'acides (méth)acryliques, les vinyllactames tels que la vinylpyrrolidone et le vinylcaprolactame, et les esters vinyliques.
   Ainsi, parmi ces copolymères conditionneurs cationiques de la famille (1), on peut citer :
   - les copolymères d'acrylamide et de méthacrylate de diméthylaminoéthyle quaternisé avec du sulfate de diméthyle ou avec un halogénure de diméthyle, tels que Hercofloc disponible auprès de la société Hercules ;
   - les copolymères d'acrylamide et de chlorure de méthacryloyloxyéthyltriméthylammonium qui sont divulgués, par exemple, dans le document EP-A-080 976, dont la divulgation concernant les polymères cationiques est incorporée ici par référence, et vendus par exemple sous le nom Bina Quat P 100 par la société Ciba-Geigy ;
   - les copolymères d'acrylamide et de méthosulfate de méthacryloyloxyéthyltriméthylammonium, tels que Reten disponible auprès de la société Hercules ;
   - éventuellement les copolymères vinylpyrrolidone/(méth)acrylate de dialkyl-aminoalkyle, qui sont divulgués, par exemple, dans les brevets français 2 077 143 et 2 393 573, et vendus, par exemple, sous le nom « Gafquat » par la société ISP, tels que, par exemple, « Gafquat 734 » ou « Gafquat 755 », ou bien les produits dénommés « Copolymère 845, 958 et 937 » ;
   - les terpolymères méthacrylate de diméthylaminoéthyle/vinylcaprolactame/vinylpyrrolidone tels que le produit vendu sous le nom Gaffix VC 713 par la société ISP;
   - les copolymères vinylpyrrolidone/ méthacrylamidopropyldiméthylamine vendus en particulier sous le nom Styleze CC 10 par ISP, et
   - le copolymère vinylpyrrolidone/diméthylamino-propylméthacrylamide quaternisé, tel que le produit vendu sous le nom « Gafquat HS 100 » par la société ISP.
(2) les dérivés d'éther de cellulose contenant des groupements ammonium quaternaires décrits dans le brevet français 1 492 597. Des exemples incluent les polymères vendus sous les noms « JR » (JR 400, JR 125 et JR 30M) ou « LR » (LR 400 et LR 30M) par la société Amerchol. Ces polymères sont également définis dans le dictionnaire CTFA comme des ammoniums quaternaires d'hydroxyéthylcellulose ayant réagi avec un époxyde substitué par un groupement triméthylammonium.
(3) les dérivés de cellulose cationiques tels que les copolymères de cellulose et les dérivés de cellulose greffés avec un monomère hydrosoluble d'ammonium quaternaire, et divulgués notamment dans le brevet U.S. 4 131 576. Des exemples incluent des hydroxyalkylcelluloses, par exemple les hydroxyméthyl-, hydroxyéthyl-, et hydroxypropyl-celluloses greffées notamment avec un sel de méthacryloyléthyltriméthylammonium, de méthacrylamidopropyltriméthylammonium, ou de diallyldiméthylammonium.
   Les produits commercialisés répondant à cette définition sont plus particulièrement les produits vendus sous le nom « Celquat L 200 » et «Celquat H 100 » par la société National Starch.
(4) les chitosanes ou leurs sels. Les sels pouvant être utilisés sont notamment l'acétate, le lactase, le glutamate, le gluconate ou le pyrrolidone-carboxylate de chitosane.
   Parmi ces composés, on peut citer le chitosane ayant un degré de déacétylation de 90,5 % en poids, vendu sous le nom Kytan Crude Standard par la société Aber Technologies, et le pyrrolidone-carboxylate de chitosane vendu sous le nom Kytamer PC par la société Amerchol.
(5) les polysaccharides quaternisés, divulgués plus particulièrement dans les brevets U.S. n° 3 589 578 et 4 031 307, tels que les gommes de guar comprenant des groupements cationiques trialkylammonium. Des exemples incluent des gommes de guar modifiées par un sel (*p*. *ex*., le chlorure) de 2,3-époxypropyltriméthylammonium.
   De tels produits sont vendus notamment sous les noms commerciaux Jaguar C13 S, Jaguar C 15, Jaguar C 17, et Jaguar C162 par la société Rhodia Chimie.
(6) les copolymères comprenant des motifs pipérazinyle des groupements divalents alkylène ou hydroxyalkylène à chaînes droite et ramifiée, éventuellement interrompus par un ou plusieurs atomes d'oxygène, atomes de soufre, atomes d'azote, ou par des cycles aromatiques, et des cycles hétérocycliques. Des exemples incluent des produits d'oxydation et de quaternisation de ces copolymères. De tels polymères sont décrits notamment dans les brevets français 2 162 025 et 2 280 361.
(7) les polyaminoamides hydrosolubles, qui peuvent être préparés par polycondensation d'un composé acide avec une polyamine. Ces polyaminoamides peuvent être réticulés par au moins un composé choisi parmi les épihalohydrines, les diépoxydes, les dianhydrides, les dianhydrides insaturés, les dérivés bis-insaturés, les bis-halohydrines, les bis-azétidiniums, les bis-haloacyldiamines, les bis-halogénures d'alkyle, et les oligomères résultant de la réaction d'un composé difonctionnel, réactif vis-à-vis d'un composé choisi parmi les bis-halohydrines, les bis-azétidiniums, les bis-haloacyldiamines, les bis-halogénures d'alkyle, les épihalohydrines, les diépoxydes, et les dérivés bis-insaturés. L'agent réticulant peut être utilisé dans des proportions allant d'environ 0,025 à environ 0,35 mole par groupement amine du polyaminoamide. Dans un mode de mise en oeuvre, ces polyaminoamides sont alkylés et/ou, s'ils contiennent une ou plusieurs fonctions amines tertiaires, quaternisés. De tels polymères sont décrits notamment dans les brevets français 2 252 840 et 2 368 508.
(8) les dérivés de polyaminoamides résultant de la condensation de polyalkylènes-polyamines avec des acides polycarboxyliques, suivie d'une alkylation par des agents difonctionnels. Des exemples incluent des polymères acide adipique/dialkylaminohydroxyalkyldialkylènetriamine dans lesquels les groupements alkyle sont des groupements alkyle sont des groupements alkyle en C₁-C₄ (tels que méthyle, éthyle, et propyle), tels que les polymères acide adipique/diméthylaminohydroxypropyldiéthylènetriamine vendus sous le nom « Cartaretine F, F4 et F8 » par la société Sandoz. De tels polymères sont décrits notamment dans le brevet français 1 583 363.
(9) les polymères obtenus par réaction d'une polyalkylène-polyamine contenant deux groupements amines primaires et au moins un groupement amine secondaire, avec un acide dicarboxylique choisi parmi les acides diglycoliques et les acides dicarboxyliques aliphatiques saturés ayant de 3 à 8 atomes de carbone. Le rapport molaire entre la polyalkylène-polyamine et l'acide dicarboxylique est compris entre environ 0,8:1 et environ 1,4:1. Le polyaminoamide en résultant est mis en réaction avec l'épichlorohydrine dans un rapport molaire d'épichlorohydrine par rapport au groupement amine secondaire du polymaminoamide compris entre environ 0,5:1 et environ 1,8:1. Dé tels polymères sont décrits notamment dans les brevets U.S. n° 3 227 615 et 2 961 347.
   Des polymères de ce type sont vendus notamment sous le nom «Hercosett 57 » par la société Hercules Inc., ou bien sous le nom « PD 170 » ou «Delsette 101 » par la société Hercules dans le cas du copolymère acide adipique/époxypropyl/diéthylènetriamine.
(10) les cyclopolymères d'alkyldiallylamine et/ou de dialkyldiallylammonium, tels que les homopolymères ou copolymères contenant, comme constituant principal de la chaîne, des motifs choisis parmi les formules (IV) et/ou (V) : 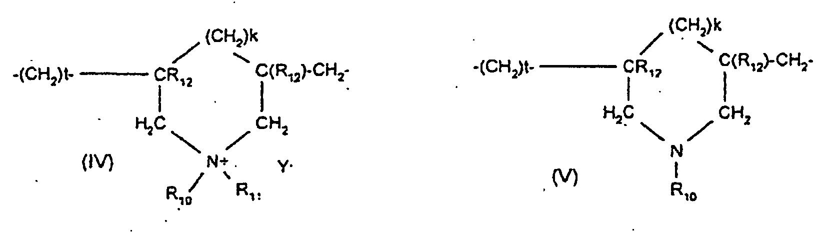 dans lesquelles k et t sont soit 0 soit 1 et la somme k + t est égale à 1; chaque R₁₂ est choisi indépendamment parmi l'hydrogène et les groupements méthyle; R₁₀ et R₁₁ sont choisis indépendamment parmi les groupements alkyle ayant de 1 à 8 atomes de carbone (tels que de 1 à 4 atomes de carbone), les groupements hydroxyalkyle en C₁-C₅ et les groupements amidoalkyle en C₁-C₄, ou R₁₀ et R₁₁, pris ensemble avec l'atome d'azote auquel ils sont liés, peuvent former un groupement hétérocyclique tel que pipéridyle et morpholinyle; Y⁻ est choisi indépendamment parmi les anions tels que les anions bromure, chlorure, acétate, borate, citrate, tartrate, bisulfate, bisulfite, sulfate, et phosphate. Ces polymères sont décrits notamment dans le brevet français 2 080 759 et dans son certificat d'addition 2 190 406.
   Parmi les polymères définis ci-dessus, on peut citer plus particulièrement l'homopolymère de chlorure de diméthyldiallylammonium vendu sous le nom « Merquat 100 » par la société Calgon (ses homologues de faible masse moléculaire moyenne en poids) et les copolymères de chlorure de diallyldiméthylammonium et d'acrylamide, vendus sous le nom « Merquat 550 ».
(11) les polymères de diammonium quaternaire contenant des motifs récurrents répondant à la formule (VI) : 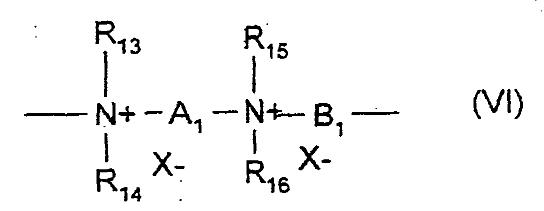 dans laquelle R₁₃, R₁₄, R₁₅ et R₁₆ sont choisis indépendamment parmi des groupements aliphatique en C₁₋C₂₀, alicyclique en C₁₋C₂₀ ou arylaliphatique en C₁₋C₂₀, et des groupements hydroxyalkyle aliphatiques inférieurs ; ou bien R₁₃ R₁₄, R₁₅ et R₁₆, ensemble ou séparément, constituent avec les atomes d'azote auxquels ils sont liés des hétérocycles contenant éventuellement un second hétéroatome autre que l'azote; ou bien R₁₃, R₁₄, R₁₅ et R₁₆ sont choisis indépendamment parmi des groupements alkyle en C₁₋C₆ linéaires et ramifiés, substitués par au moins un groupement choisi parmi les groupements nitriles, esters, acyles, amides, -CO-O-R₁₇-D, et des groupements -CO-NH-R₁₇-D, où R₁₇ est un alkylène et D est un groupement ammonium quaternaire; A₁ et B₁ sont choisis indépendamment parmi des groupements polyméthylène linéaires et ramifiés, saturés et insaturés, contenant de 2 à 20 atomes de carbone et pouvant contenir, liés à ou intercalés dans la chaîne principale, au moins un groupement choisi parmi les cycles aromatiques, l'oxygène, le soufre, les sulfoxydes, les sulfones, les disulfures, les amino, les alkylamino, les hydroxy, les ammonium quaternaires, les uréido, les amides et les esters ; X⁻ est choisi indépendamment parmi les anions issus des acides minéraux ou organiques. Dans un mode de mise en oeuvre, X⁻ est un anion tel que le chlorure et le bromure.
   A₁, R₁₃, et R₁₅ peuvent former, avec les deux atomes d'azote auxquels ils sont liés, un cycle pipérazinique.
   De manière alternative, A₁ est choisi parmi les groupements alkylène ou hydroxyalkylène linéaires et ramifiés, saturés et insaturés, et B₁ est choisi parmi des groupements (CH₂)ₙ-CO-E-OC-(CH₂)ₙ- dans lesquels n varie de 1 à 6 et E est choisi parmi :
   a) des restes de glycol de formule: -O-Z-O-, où Z est choisi parmi des groupements hydrocarbonés linéaires et ramifiés et des groupements répondant à l'une des formules suivantes.:

      -(CH₂₋CH₂-O)ₓ-CH₂-CH₂-

      et

      -[CH₂-CH(CH₃)-O]_{y}-CH₂-CH(CH₃)-

      où x et y sont des entiers allant indépendamment de 1 à 4 et représentant un degré de polymérisation défini et unique ou un nombre quelconque de 1 à 4 et représentant un degré de polymérisation moyen ;
   b) des restes diamines bis-secondaires, tels qu'un dérivé de pipérazine ;
   c) des restes de diamines bis-primaires, de formule : -NH-Y-NH-, où Y est choisi parmi des groupements hydrocarbonés linéaires et ramifiés et-CH₂-CH₂-S-S-CH₂-CH₂-; et
   d) des groupements uréylène de formule :

      -NH-CO-NH-.

   Ces polymères ont généralement une masse moléculaire moyenne en nombre allant d'environ 1000 à environ 100 000. Des exemples de ces polymères de ce type sont décrits dans les brevets U.S. n° 2 273 780, 2375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945, et 4027 020.
   Dans un mode de mise en oeuvre, les polymères conditionneurs cationiques de la famille (11) sont constitués de motifs récurrents répondant à la formule : 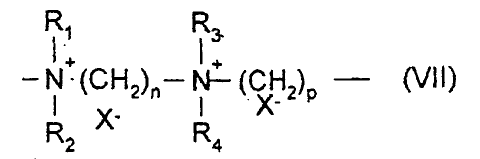 dans laquelle R₁, R₂, R₃, et R₄ sont choisis indépendamment parmi le radical alkyle en C₁-C₄ et hydroxyalkyle en C₁-C₄, n et p sont des entiers allant indépendamment d'environ 2 à environ 20 et X⁻ est un anion choisi parmi les acides minéraux et organiques.
   Dans un mode de mise en oeuvre, R₁, R₂, R₃, et R₄ sont choisis parmi des groupements méthyle; n = 3; p = 6; et X = Cl, dénommé chlorure d'hexadiméthrine selon la nomenclature de l'INCI (CTFA).
(12) les polymères de polyammonium quaternaires constitués de motifs de formule (VIII) : 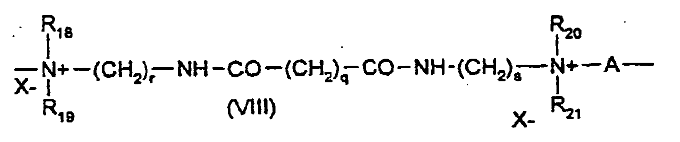 dans laquelle R₁₈, R₁₉, R₂₀, et R₂₁ sont choisis indépendamment parmi l'hydrogène, les groupements méthyle, éthyle, propyle, □-hydroxyéthyle, β-hydroxypropyle, et -CH₂CH₂(OCH₂CH₂)ₚOH, où p est un entier allant de 0 à 6 sous réserve que R₁₈, R₁₉, R₂₀, et R₂₁ ne sont pas simultanément l'hydrogène ; r et s sont des entiers allant indépendamment de 1 à 6 ; q est un entier allant de0 à 34 ; X⁻ est choisi parmi les anions, tels que les halogénures ; A est choisi parmi des groupements dihalogénures tels que - CH₂-CH₂-O-CH₂-CH₂-.
   De tels composés sont notamment décrits dans la demande de brevet EP-A-122 324. Parmi ces produits, on peut citer par exemple « Mirapol® A 15 », « Mirapol® AD1 », « Mirapol® AZ1 » et « Mirapol® 175 » vendus par la société Miranol.
(13) les polymères quaternaires de vinylpyrrolidone et de vinylimidazole tels que, par exemple, les produits vendus sous les noms Luviquat® TFC, FC 905, FC 550 et FC 370 par la société BASF.
(14) les polyamines telles que Polyquart® H vendu par Henkel référencées sous le nom de « Polyethylene glycol (15) tallow polyamine » dans le dictionnaire CTFA.
(15) les polymères réticulés ou non réticulés de sels de méthacryloyloxyalkyl(C₁-C₄)trialkyl(C₁-C₄)ammonium tels que les polymères obtenus par homopolymérisation du méthacrylate de diméthylaminoéthyle quaternisé par le chlorure de méthyle ou par copolymérisation de l'acrylamide avec le méthacrylate de diméthylaminoéthyle quaternisé par un halogénure (tel que chlorure) de méthyle, l'homopolymérisation ou la copolymérisation étant suivie d'une réticulation par un composé à insaturation oléfinique, notamment le méthylènebisacrylamide. Dans un mode de mise en oeuvre, on utilise un copolymère réticulé acrylamide/chlorure de méthacryloyloxyéthyl-triméthylammonium (20/80 en poids) sous forme d'une dispersion contenant 50 % en poids dudit copolymère dans de l'huile minérale. Cette dispersion est vendue sous le nom « Salcare® SC 92 » par la société Ciba.. Dans un autre mode de mise en oeuvre, on peut également utiliser un homopolymère réticulé du chlorure de méthacryloyloxyéthyl-triméthylammonium contenant environ 50 % en poids de l'homopolymère dans de l'huile minérale ou dans un ester liquide. Ces dispersions sont vendues sous les noms « Salcare® SC 95 » et « Salcare® SC 96 » par la société Ciba.

D'autres polymères conditionneurs cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Dans un mode de mise en oeuvre, les polymères conditionneurs cationiques sont choisis parmi les dérivés d'éther de cellulose quaternaires tels que les produits vendus sous le nom « JR 400 » par la société Amerchol; la gomme de guar quaternisée telle que les produits vendus sous le nom « Jaguar C 13 S » par la société Rhodia ; les cyclopolymères cationiques, notamment les homopolymères ou les copolymères du chlorure de diméthyldiallylammonium, vendus sous les noms « Merquat 100 », « Merquat 550 » et « Merquat S » par la société Calgon, les polymères quaternaires de vinylpyrrolidone et de vinylimidazole; les polymères non réticulés et réticulés de sels de méthacryloyloxyalkyl(C₁-C₄)trialkyl(C₁-C₄)ammonium tels que les produits vendus sous le nom « Salcare SC 96 » par la société NALCO ; et des mélanges de ceux-ci. Selon un mode de réalisation particulièrement préférés, les compositions selon l'invention comprennent au moins un polymères cationiques choisi parmi les homopolymères ou les copolymères du chlorure de diméthyldiallylammonium.

Selon l'invention, le(s) polymère(s) cationique(s) peut (peuvent) représenter d'environ 0,001 % à environ 20 % en poids, par exemple d'environ 0,01 % à environ 10 % en poids, encore par exemple d'environ 0,1 % à environ 3 % en poids, par rapport au poids total de la composition finale.

Les polymères anioniques pouvant être utilisés selon la présente invention sont des polymères comprenant des groupements dérivés de l'acide carboxylique, sulfonique et/ou phosphorique et ayant un poids moléculaire moyen en poids allant d'environ 500 à environ 5 000 000.
(1) Les groupements carboxyliques peuvent être apportés par des monomères monoacides ou diacides carboxyliques insaturés tels que ceux répondant à la formule : 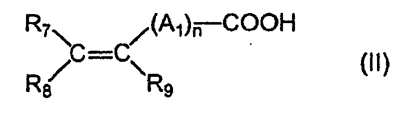 dans laquelle n est un entier de 0 à 10 ; A₁ désigne un groupement méthylène et lorsque n est supérieur à 1, chaque A₁ est représenté indépendamment par-LCH₂-, où L est choisi parmi une liaison de valence et des hétéroatomes tels que l'oxygène et le soufre ; R₇ est choisi parmi l'hydrogène, des groupements phényle, et des groupements benzyle ; R₈ est choisi parmi l'hydrogène, des groupements alkyle inférieur, et des groupements carboxy ; et R₉ est choisi parmi l'hydrogène, des groupements alkyle inférieur, des groupements -CH₂-COOH, des groupements phényle et des groupements benzyle.
   Comme défini ici, un groupement alkyle inférieur désigne un groupement ayant de 1 à 4 atomes de carbone, tels que méthyle et éthyle.
   Les polymères anioniques comprenant des groupements carboxy selon l'invention peuvent être choisis parmi :
   A) Les homopolymères et les copolymères d'acides (méth)acryliques ou les sels (méth)acryliques, et notamment les produits vendus sous les noms Versicol E ou K par la société Allied Colloid et Ultrahold par la société BASF, les copolymères d'acide acrylique et d'acrylamide vendus sous la forme de leur sel de sodium sous les noms Reten 421, 423 ou 425 par la société Hercules, et les sels de sodium des acides polyhydroxycarboxyliques.
   B) Les copolymères d'acide (méth)acrylique avec un monomère monoéthylénique tel que l'éthylène, le styrène, les esters vinyliques et les esters d'acide (méth)acrylique, éventuellement greffés sur un polyalkylèneglycol tel que le polyéthylèneglycol, et éventuellement réticulés. De tels polymères sont divulgués en particulier dans le brevet français 1 222 944 et la demande allemande 2 330 956. Les copolymères de ce type comprenant dans leur chaîne un motif acrylamide éventuellement N-alkylé et/ou hydroxyalkylé tels que divulgués notamment dans les demandes de brevets luxembourgeois 75370 et 75371 vendus sous le nom Quadramer par la société American Cyanamid. On peut également citer les copolymères d'acide acrylique et de méthacrylate d'alkyle en C₁-C₄ et les terpolymères de vinylpyrrolidone, d'acide acrylique, et de méthacrylate d'alkyle en C₁-C₂₀, par exemple le méthacrylate de lauryle, tels que Acrylidone LM disponible auprès de la société ISP, et les terpolymères d'acide méthacrylique/acrylate d'éthyle/acrylate de tert-butyle tels que le produit vendu sous le nom Luvimer 100 P par la société BASF.
   C) Les copolymères dérivés d'acide crotonique tels que ceux comprenant dans leur chaîne des motifs acétate ou propionate de vinyle et éventuellement d'autres monomères tels que les esters (méth)allyliques, les éthers vinyliques et les esters vinyliques d'acides carboxyliques saturés, linéaires et ramifiés à longue chaîne hydrocarbonée, tels que ceux comprenant au moins 5 atomes de carbone, ces polymères pouvant éventuellement être greffés et réticulés ; ou encore les esters vinyliques et (méth)allyliques d'un acide carboxylique α- ou β-cyclique. De tels polymères sont divulgués entre autres dans les brevets français 1 222 944, 1 580 545, 2 265 782, 2 265 781, 1 564 110 et 2 439 798. Des produits commerciaux entrant dans cette classe sont les Resin 28-29-30, 26-13-14, et 28-13-10 vendues par la société National Starch.
   D) Les copolymères issus d'acides ou d'anhydrides carboxyliques monoinsaturés en C₄-C₈ choisis parmi :
      - les copolymères comprenant des motifs issus (i) d'au moins un monomère choisi parmi les acides maléique, fumarique, et itaconique et leurs anhydrides et (ii) d'au moins un monomère choisi parmi les esters vinyliques, les éthers vinyliques, les halogénures vinyliques, les dérivés phénylvinyliques, les acides acryliques, et les esters d'acide acrylique, les fonctions anhydrides de ces copolymères étant éventuellement monoestérifiées ou monoamidifiées. De tels polymères sont divulgués notamment dans les brevets U.S. n° 2 047 398, 2 723 248, et 2 102 112 et GB 839 805 et notamment ceux vendus sous les noms Gantrez AN ou ES par la société ISP.
      - les copolymères comprenant des motifs issus (i) d'au moins un monomère choisi parmi les anhydrides maléique, citraconique, et itaconique et (ii) d'au moins un monomère choisi par les esters (méth)allyliques comprenant éventuellement dans leur chaîne au moins un motif issu de groupements choisis parmi les groupements (méth)acrylamide, α-oléfine, ester (méth)acrylique, acide (méth)acrylique, et vinylpyrrolidone. Les fonctions anhydrides de ces copolymères sont éventuellement monoéstérifiées ou monoamidifiées.

      Ces polymères sont par exemple divulgués dans les brevets français 2 350 384 et 2 357 241.
   E) les polyacrylamides comprenant des groupements carboxylates.
(2) Les polymères anioniques comprenant des groupements sulfoniques peuvent être choisis parmi les polymères comprenant des motifs, tels que ceux issus des acides vinylsulfonique, styrènesulfonique, naphthalènesulfonique, et acrylamidoalkylsulfonique et leurs dérivés. Ces polymères peuvent être choisis parmi :
   - les sels d'acide polyvinylsulfonique ayant un poids moléculaire moyen en poids allant d'environ 1000 à environ 100 000, ainsi que les copolymères issus d'au moins un comonomère insaturé tel que les acides (méth)acryliques, leurs esters, les acrylamides, leurs dérivés, les éthers vinyliques et la vinylpyrrolidone ;
   - les sels de l'acide polystyrènesulfonique, les sels de sodium ayant un poids moléculaire moyen en poids allant d'environ 100 000 à environ 500 000, vendus respectivement sous les noms Flexan 500 et Flexan 130 par National Starch. Ces composés sont divulgués dans le brevet FR 2 198 719 ;
   - les sels d'acides polyacrylamidesulfoniques, dont ceux cités dans le brevet U.S. n° 4 128 631, et plus particulièrement l'acide polyacrylamidoéthylpropanesulfonique vendu sous le nom Cosmedia Polymer HSP 1180 par Henkel.

Dans un mode de mise en oeuvre, les polymères anioniques sont choisis parmi les copolymères d'acide acrylique tels que le terpolymère acide acrylique/acrylate d'éthyle/N-tert-butylacrylamide vendu sous le nom Ultrahold Strong par la société BASF ; les copolymères issus d'acide crotonique tels que les terpolymères d'acétate de vinyle/tert-butylbenzoate de vinyle/acide crotonique et les terpolymères acide crotonique/acétate de vinyle/néododécanoate de vinyle vendus sous le nom Resin 28-29-30 par la société National Starch ; les polymères issus d'au moins un monomère choisi parmi les acides maléique, fumarique, et itaconique et leurs anhydrides et également d'au moins un monomère choisi parmi les esters vinyliques, les éthers vinyliques, les halogénures vinyliques, les dérivés phénylvinyliques, l'acide acrylique, et les esters d'acide acrylique, tels que le copolymère méthylvinyléther/anhydride maléique monoestérifié vendu sous le nom Gantrez ES 425 par la société ISP ; les copolymères d'acide méthacrylique et de méthacrylate de méthyle vendus sous le nom Eudragit L par la société Rohm Pharma ; le copolymère d'acide méthacrylique et d'acrylate d'éthyle vendu sous le nom Luvimer MAEX ou MAE par la société BASF ; le copolymère acétate de vinyle/acide crotonique vendu sous le nom Luviset CA 66 par la société BASF; et le copolymère acétate de vinyle/acide crotonique greffé par du polyéthylèneglycol vendu sous le nom Aristoflex A par la société BASF.

Dans un autre mode de mise en oeuvre, les polymères anioniques sont choisis parmi le copolymère méthylvinyléther/anhydride maléique monoestérifié vendu sous le nom Gantrez ES 425 par la société ISP ; le terpolymère acide acrylique/acrylate d'éthyle/N-tert-butylacrylamide vendu sous le nom Ultrahold Strong par la société BASF; les copolymères d'acide méthacrylique et de méthacrylate de méthyle vendus sous le nom Eudragit L par la société Rohm Pharma ; les terpolymères acétate de vinyle/tert-butylbenzoate de vinyle/acide crotonique et les terpolymères acide crotonique/acétate de vinyle/néododécanoate de vinyle vendus sous le nom Resin 28-29-30 par la société National Starch; le copolymère d'acide méthacrylique et d'acrylate d'éthyle vendu sous le nom Luvimer MAEX ou MAE par la société BASF ; et le terpolymère vinylpyrrolidone/acide acrylique/méthacrylate de lauryle vendu sous le nom Acrylidone LM par la société ISP.

Les polymères amphotères utilisables conformément à l'invention peuvent être choisis parmi les polymères comprenant des motifs X et Y, répartis statistiquement dans la chaîne polymère, où le motif X est choisi parmi les motifs issus d'au moins un monomère comprenant au moins une fonction basique, notamment un atome d'azote basique, et où le motif Y est choisi parmi les motifs issus d'au moins un monomère acide comprenant au moins un groupement choisi parmi les groupements carboxy et les groupements sulfo, ou bien où chaque motif X et Y est choisi indépendamment parmi les groupements issus de monomères zwitterioniques de carboxybétaïne et de sulfobétaïne. Dans un autre mode de mise en oeuvre, les polymères amphotères pouvant être utilisés conformément à l'invention peuvent être choisis parmi les polymères comprenant des motifs X et Y, chaque motif X et Y est choisi indépendamment parmi au moins une chaîne polymère cationique comprenant au moins un groupement choisi parmi des groupements amines primaires, des groupements amines secondaires, des groupements amines tertiaires, et des groupements amines quaternaires, dans laquelle au moins l'un des groupements amines comprend un groupement choisi parmi les groupements carboxy et sulfo reliés par l'intermédiaire d'un groupement hydrocarboné, ou bien les motifs X et Y, identiques ou différents, font partie d'une chaîne d'au moins un polymère comprenant un motif α,β-dicarboxy éthylène, où au moins l'un des groupements carboxy a été mis en réaction avec une polyamine comprenant au moins un groupement choisi parmi des groupements amines primaires et secondaires.

Dans un mode de mise en oeuvre, les polymères amphotères répondant à la définition donnée ci-dessus sont choisis parmi les polymères suivants:
(1) les polymères résultant de la copolymérisation d'un monomère issu d'un composé vinylique portant un groupement carboxy tel que les acides (méth)acryliques, les acides maléiques, et les acides α-chloracryliques et d'un monomère basique issu d'un composé vinylique substitué comprenant au moins un atome basique tel que le dialkylaminoalkyl-(méth)acrylate et le dialkylaminoalkyl-(méth)acrylamide. De tels composés sont divulgués dans le brevet U.S. n° 3 836 537.
(2) les polymères comprenant des motifs issus :
   a) d'au moins un monomère choisi parmi les (méth)acrylamides substitués sur l'azote par un groupement alkyle,
   b) d'au moins un comonomère acide comprenant au moins un groupement carboxy réactif et
   c) d'au moins un comonomère basique tel que des esters comprenant au moins un substituant choisi parmi des substituants amines primaires, secondaires, tertiaires et quaternaires des acides (méth)acryliques, et le produit de quaternisation du méthacrylate de diméthylaminoéthyle avec le sulfate de diméthyle ou diéthyle.

   Ledit au moins un monomère (méth)acrylamide N-substitué cité dans (a) est choisi plus particulièrement parmi les (méth)acrylamides N-substitués dans lesquels les groupements alkyle comprennent de 2 à 12 atomes de carbone, tels le N-éthylacrylamide, le N-tert-butylacrylamide, le N-tert-octylacrylamide, le N-octylacrylamide, le N-décylacrylamide, le N-dodécylacrylamide, et les méthacrylamides correspondants.
   Ledit au moins un comonomère acide cité dans (b) est choisi plus particulièrement parmi les acides (méth)acryliques, les acides crotoniques, les acides itaconiques, les acides maléiques, les acides fumariques, les monoesters d'alkyle en C₁-C₄ d'acide maléique, les monoesters d'alkyle en C₁-C₄ d'acide fumarique, les monoesters d'alkyle en C₁-C₄ d'anhydride maléique, et les monoesters d'alkyle en C₁-C₄ d'anhydride fumarique.
   Ledit au moins un comonomère basique cité dans (c) est choisi plus particulièrement parmi les méthacrylates d'aminoéthyle, de butylaminoéthyle, de N,N'-diméthylaminoéthyle, et de N-tert-butylaminoéthyle.
   Dans un mode de mise en oeuvre, le polymère amphotère est choisi parmi les copolymères dont le nom CTFA (4e éd., 1991) est octylacrylamide/acrylates/butylaminoethyl methacrylate copolymer, tels que les produits vendus sous le nom Amphomer ou Lovocryl 47 par la société National Starch.
(3) les polyaminoamides réticulés et alkylés partiellement ou totalement issus de polyaminoamides de formule générale : 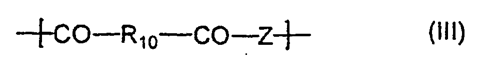 dans laquelle R₁₀ représente un groupement divalent issu d'un acide dicarboxylique saturé, d'un acide aliphatique mono- ou dicarboxylique comprenant une double liaison éthylénique, d'un ester d'un alcanol inférieur (ayant de 1 à 6 atomes de carbone) de ces acides, ou d'un groupement issu de l'addition de l'un quelconque desdits acides avec une amine bisprimaire ou bis-secondaire ; et Z désigne un groupement polyalkylène-polyamine bisprimaire, mono- ou bis-secondaire et représente par exemple :
   a) dans les proportions d'environ 60 % en mole à 100 % mole, le groupement: 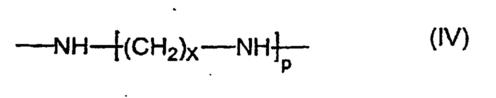 où x=2 et p=2 ou 3, ou bien x=3 et p=2 et où ce groupement est issu de diéthylènetriamine, de triéthylènetétraamine, ou de dipropylènetriamine ;
   b) dans les proportions de 0 % en mole à environ 40 % en mole, le groupement (IV) ci-dessus, dans lequel x=2 et p=1 et qui dérive de l'éthylènediamine, ou le groupement issu de la pipérazine ; 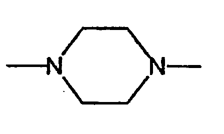
   c) dans les proportions de 0 % en mole à environ 20 % en mole, le groupement -NH-(CH₂)₆-NH- issu d'hexaméthylènediamine, ces polyaminoamines étant réticulées par addition d'un agent réticulant bifonctionnel choisi parmi les épihalohydrines, les diépoxydes, les dianhydrides et les dérivés bis-insaturés, à l'aide d'environ 0,025 mole à environ 0,35 mole d'agent réticulant par groupement amine du polyaminoamide et alkylées par l'action d'acide acrylique, d'acide chloroacétique ou d'une alcanesultone, ou leurs sels.

   Dans un mode de mise en oeuvre, les acides carboxyliques saturés sont choisis parmi les acides ayant de 6 à 10 atomes de carbone, tels que l'acide adipique, l'acide 2,2,4-triméthyladipique, l'acide 2,4,4-triméthyladipique, l'acide téréphtalique, et les acides à double liaison éthylénique comme par exemple l'acide acrylique, l'acide méthacrylique et l'acide itaconique.
   Dans un mode de mise en oeuvre, les alcanesultones utilisées dans l'alkylation sont choisies parmi la propanesultone et la butanesultone et les sels des agents d'alkylation sont choisis parmi les sels de sodium et de potassium.
(4) les polymères comprenant des motifs zwitterioniques de formule: 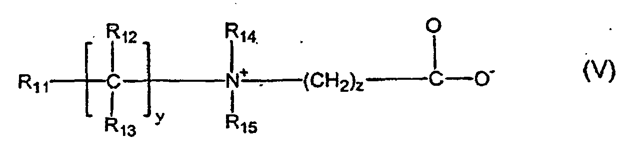 dans laquelle R₁₁ est choisi parmi les groupements insaturés polymérisables tels que des groupements (méth)acrylate et (méth)acrylamide ; y et z sont choisis indépendamment des entiers de 1 à 3 ; R₁₂ et R₁₃ sont choisis indépendamment parmi l'hydrogène, des groupements méthyle, des groupements éthyle et des groupements propyle ; R₁₄ et R₁₅ sont choisis indépendamment parmi l'hydrogène et les groupements alkyle, où la somme des atomes de carbone dans R₁₄ et R₁₅ est inférieure ou égale à 10.
   Les polymères comprenant de tels motifs peuvent également comprendre des motifs issus de monomères non zwitterioniques, tels que le (méth)acrylate de diméthylaminoéthyle, le (méth)acrylate de diéthylaminoéthyle, les (méth)acrylates d'alkyle, les (méth)acrylamides, et les acétates de vinyle.
   A titre d'exemple, on peut citer le copolymère méthacrylate de méthyle/diméthylcarboxyméthylammonio-éthyl-méthacrylate de méthyle tel que le produit vendu sous le nom Diaformer Z301 par la société Sandoz.
(5) les polymères issus du chitosane comprenant des motifs monomères répondant aux formules suivantes : 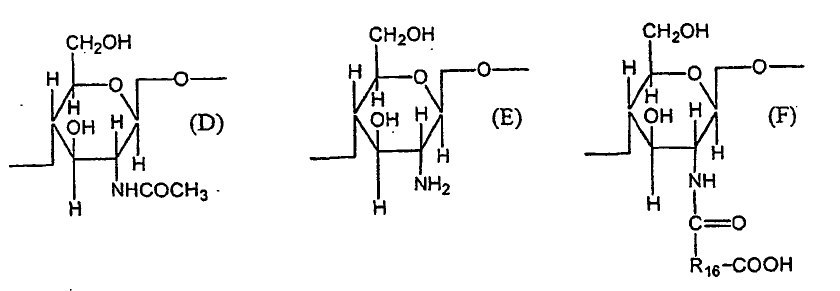 le motif D étant présent dans des proportions allant de 0 % à environ 30 %, le motif E dans des proportions allant d'environ 5 % à environ 50 % et le motif F dans des proportions allant d'environ 30 % à environ 90 %, étant entendu que dans ce motif F, R₁₆ représente un groupement de formule: 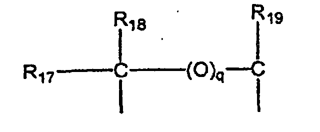 dans laquelle, si q=0, R₁₇, R₁₈ et R₁₉, identiques ou différents, sont choisis parmi l'hydrogène, des groupements méthyle, des groupements hydroxy, des groupements acétoxy, des résidus amino, des résidus monoalkylamine, et des résidus dialkylamine, éventuellement interrompus par un ou plusieurs atomes d'azote et/ou éventuellement substitués par un ou plusieurs groupements amine, hydroxy, carboxy, alkylthio, ou sulfo, et des résidus alkylthio dans lesquels le groupement alkyle porte un résidu amino, au moins l'un de R₁₇, R₁₈ et R₁₉ étant, dans ce cas, l'hydrogène ; ou si q=1, R₁₇, R₁₈ et R₁₉ représentent chacun l'hydrogène, et les sels formés par ces composés avec des bases ou des acides.
(6) Les polymères issus de la N-carboxyalkylation du chitosane, tels que le N-(carboxyméthyl)chitosane ou le N-(carboxybutyl)chitosane vendu sous le nom « Evalsan » par la société Jan Dekker.
(7) Les polymères répondant à la formule générale (VI), par exemple divulgués dans le brevet français 1.400.366. 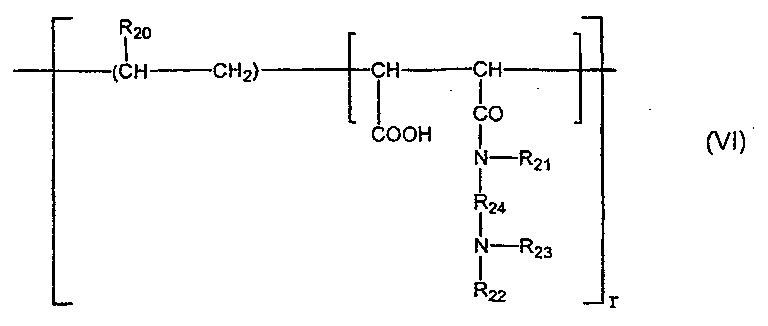 dans laquelle R₂₀ est choisi parmi l'hydrogène, les groupements CH₃O, CH₃CH₂O et phényle ; R₂₁ est choisi parmi l'hydrogène et les groupements alkyle inférieurs tels que méthyle ou éthyle ; R₂₂ est choisi parmi l'hydrogène et les groupements alkyle inférieurs tels que méthyl ou éthyle; et R₂₃ est choisi parmi les groupements alkyle inférieurs tels que méthyle ou éthyle et les groupements répondant à la formule :-R₂₄-N(R₂₂)₂, où R₂₄ est choisi parmi les groupements-CH₂-CH₂-,-CH₂-CH₂-CH₂-, et -CH₂-CH(CH₃)- et R₂₂ a les mêmes significations que ci-dessus, et les homologues supérieurs de ces groupements comprenant jusqu'à 6 atomes de carbone.
(8) Les polymères amphotères du type -D-X-D-X- choisis parmi :
   a) les polymères obtenus par la réaction de l'acide chloroacétique ou le chloroacétate de sodium avec les composés comprenant au moins un motif de formule :

      -D-X-D-X-D- (VII)

      dans laquelle D désigne un groupement 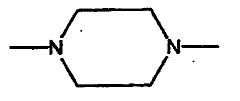 et X désigne le symbole E ou E'. E et E', identiques ou différents, désignent un groupement bivalent choisi parmi des groupements alkylène à chaîne droite et ramifiée comprenant jusqu'à 7 atomes de carbone dans la chaîne principale, qui est non substitué ou substitué par des groupements hydroxy et pouvant comprendre en outre des atomes d'oxygène, d'azote ou de soufre, ou 1 à 3 cycles aromatiques et/ou hétérocycliques ; les atomes d'oxygène, d'azote et de
      soufre étant présents sous forme de groupements éther, thioéther, sulfoxyde, sulfone, sulfonium, alkylamine ou alcénylamine, de groupements benzylamine, oxyde d'amine, ammonium quaternaire, amide, imide, alcool, ester, et/ou uréthanne.
   b) les polymères de formule :

      -D-X-D-X- (VII')

      dans laquelle D désigne un groupement 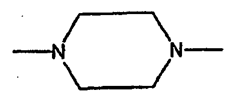 et X désigne le symbole E ou E', où X désigne E' au moins une fois, E a la signification indiquée ci-dessus et E' est un groupement bivalent choisi parmi des groupements alkylène à chaîne droite et ramifiée ayant jusqu'à 7 atomes de carbone dans la chaîne principale, qui est non substitué ou substitué par un ou plusieurs groupements hydroxy et comprenant un ou plusieurs atomes d'azote, l'atome d'azote étant substitué par une chaîne alkyle éventuellement interrompue par un atome d'oxygène et comprenant obligatoirement un ou plusieurs groupements fonctionnels carboxy et un ou plusieurs groupements fonctionnels hydroxy et où le polymère de formule VII' est bétaïnisé par réaction avec l'acide chloroacétique ou chloroacétate de sodium.
(9) Les copolymères alkyl(C₁-C₅)vinyléther/anhydride maléïque qui sont modifiés partiellement par semiamidification avec une N,N-dialkylaminoalkylamine telle que la N,N-diméthylaminopropylamine, ou par semiestérification avec une N,N-dialcanolamine. Ces copolymères peuvent également comprendre d'autres comonomères vinyliques tels que le vinylcaprolactame.

Dans un mode de mise en oeuvre, les polymères amphotères selon l'invention sont choisis dans la famille (3), comme les copolymères dont le nom CTFA (4e éd. 1991) est copolymère de méthacrylate d'octylacrylamide/d'acrylateide butylaminoéthyle, tels que les produits vendus sous les noms Amphomer, Amphomer LV 71 ou Lovocryl 47 par la société National Starch et la famille (4), tels que le copolymère de méthacrylate de méthyle/diméthyl-carboxyméthylammonio éthyl-méthacrylate de méthyle vendu par exemple sous le nom Diaformer Z301 par la société Sandoz.

Les polymères non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines vendues par la société Dow Chemical sous les noms PEOX 50 000, PEOX 200 000 et PEOX 500 000 ;
- les homopolymères d'acétate de vinyle tels que le produit vendu sous le nom Appretan EM par la société Hoechst ou le produit vendu sous le nom Rhodopas A 012 par la société Rhodia Chimie ;
- les copolymères d'acétate de vinyle et d'ester acrylique tels que le produit vendu sous le nom Rhodopas AD 310 par Rhodia Chimie ;
- les copolymères d'acétate de vinyle et d'éthylène tels que le produit vendu sous le nom Appretan TV par la société Hoechst;
- les copolymères d'acétate de vinyle et d'ester maléïque, par exemple de maléate de dibutyle tels que le produit vendu sous le nom Appretan MB Extra par la société Hoechst ;
- les copolymères de polyéthylène et d'anhydride maléïque ;
- les homopolymères d'acrylate d'alkyle et les homopolymères de méthacrylate d'alkyle tels que le produit vendu sous le nom Micropearl RQ 750 par la société Matsumoto ou le produit vendu sous le nom Luhydran A 848 S par la société BASF ;
- les copolymères d'esters acryliques tels que par exemple les copolymères de (méth)acrylates d'alkyle, tels que les produits vendus par la société Rohm & Haas sous les noms Primal AC-261 K et Eudragit NE 30D, par la société BASF sous les noms Acronal 601, Luhydran LR 8833 ou 8845, et par la société Hoechst sous les noms Appretan N 9213 ou N 9212 ;
- les copolymères d'acrylonitrile et d'un monomère non ionique choisis par exemple parmi le butadiène et les (méth)acrylates d'alkyle ; on peut citer les produits vendus sous les noms Nipol LX 531 B par la société Nippon Zeon ou ceux vendus sous le nom CJ0601 B par la société Rohm & Haas ;
- les polyuréthannes tels que les produits vendus sous les noms Acrysol RM 1020 ou Acrysol RM 2020 par la société Rohm & Haas, et les produits Uraflex XP 401 UZ et Uraflex XP 402 UZ par la société DSM Resins;
- les copolymères d'acrylate d'alkyle et d'uréthanne tels que le produit 8538-33 par la société National Starch ;
- les polyamides tels que le produit Estapor LO 11 vendu par la société Rhodia Chimie ;
- les gommes de guar non ioniques chimiquement modifiées ou non modifiées.

Les gommes de guar non ioniques non modifiées sont par exemple les produits vendus sous le nom Vidogum GH 175 par la société Unipectine et sous le nom Jaguar C par la société Rhodia Chimie.

Les gommes de guar non ioniques modifiées utilisables selon l'invention sont par exemple modifiées par des groupements hydroxyalkyle en C₁-C₆. On peut citer à titre d'exemples les groupements hydroxyméthyle, hydroxyéthyle, hydroxypropyle et hydroxybutyle.

Ces gommes de guar sont bien connues dans la technique antérieure et peuvent être préparées par exemple en faisant réagir des oxydes d'alcène correspondants tels que par exemple des oxydes de propylène, avec la gomme de guar de façon à obtenir une gomme de guar modifiée par des groupements hydroxypropyle.

De telles gommes de guar non ioniques éventuellement modifiées par des groupements hydroxyalkyle sont par exemple vendues sous les noms commerciaux Jaguar HP8, Jaguar HP60, Jaguar HP120, Jaguar DC 293, et Jaguar HP 105 par la société Rhodia Chimie et sous le nom Galactosol 4H4FD2 par la société Aqualon.

Les groupements alkyle des polymères non ioniques comprennent de 1 à 6 atomes de carbone, sauf mention contraire.

Selon l'invention, on peut également utiliser des polymères du type silicone greffée comprenant une portion polysiloxane et une portion comprenant une chaîne organique non siliconée, l'une des deux portions constituant la chaîne principale du polymère et l'autre étant greffée sur la chaîne principale. Ces polymères sont divulgués, par exemple, dans les documents EP-A-0 412 704, EP-A-0 412 707, EP-A-0 640 105, WO 95/00578, EP-A-0 582 152 et WO 93/23009 et les brevets U.S. n° 4 693 935, 4 728 571 et 4 972 037. Ces polymères sont par exemple anioniques ou non ioniques.

De tels polymères sont par exemple des copolymères susceptibles d'être obtenus par polymérisation radicalaire à partir du mélange de monomères comprenant :
a) environ 50 % à environ 90 % en poids d'acrylate de tert-butyle ;
b) 0 % à environ 40 % en poids d'acide acrylique ;
c) environ 5 % à environ 40 % en poids de macromère siliconé de formule: 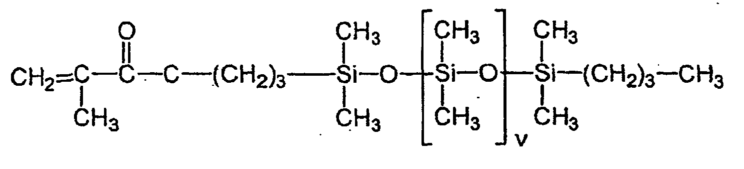
dans laquelle v est un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'une chaîne de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'une chaîne de raccordement de type thiopropylène, des motifs polymères du type poly(méth)acrylate d'isobutyle.

Il est également possible d'utiliser, comme polymères, des polyuréthannes fonctionnalisés ou non et siliconés ou non.

Des exemples de polyuréthannes utiles incluent ceux divulgués dans les brevets EP 0 751 162, EP 0 637 600, FR 2 743 297, EP 0 648 485, EP 0 656 021, WO 94/03510 et EP 0 619 111.

Dans un autre mode de mise en oeuvre, les polymères peuvent être utilisés sous forme solubilisée ou peuvent être sous forme de dispersions de particules solides ou liquides (latex ou pseudolatex).

Dans un mode de mise en oeuvre, l'au moins un constituant est choisi parmi les tensioactifs anioniques, amphotères, non ioniques, cationiques, et des mélanges de ceux-ci. Dans un mode de mise en oeuvre, l'au moins un tensioactif est présent en une quantité allant d'environ 0,1 % à environ 60 % en poids, par exemple d'environ 1 % à environ 40 %, en outre d'environ 5 % à environ 30 %, par rapport au poids total de la composition. Dans un autre mode de mise en oeuvre, la composition est un shampooing dans lequel l'au moins un tensioactif est présent en une quantité allant d'environ 5 % à environ 30 % par rapport au poids total de la composition. Dans encore un autre mode de mise en oeuvre, la composition est un conditionneur dans lequel le tensioactif est présent en une quantité allant d'environ 0,1 % à environ 15 % par rapport au poids total de la composition et de préférence de 0,5 à 5% en poids. Dans un mode de mise en oeuvre, la composition est un conditionneur dans lequel l'au moins un tensioactif est un tensioactif cationique.

Les tensioactifs représentatifs convenant à la mise en oeuvre de la présente invention sont par exemple les suivants :

### (i) Tensioactif(s) anionique(s) :

Dans le contexte de la présente invention, la nature du tensioactif anionique n'est pas d'une importance critique. Ainsi, des exemples de tensioactifs anioniques pouvant être utilisés seuls ou en mélange incluent les sels (tels que les sels alcalins, notamment les sels de sodium, les sels d'ammonium, les sels d'amine, les sels d'aminoalcool et les sels de magnésium) de composés (tels que les alkylsulfates, les alkyléthersulfates, les alkylamidoéthersulfates, les alkylarylpolyéthersulfates, les monoglycéride-sulfatés, les alkylsufonates, les alkylphosphates, les alkylamidesulfonates, les alkylarylsufonates, les α-oléfine-sulfonates, les paraffine-sulfonates, les alkylsulfosuccinates, les alkyléthersulfosuccinates, les alkylamidesulfosuccinates, les alkylsulfosuccinamates, les alkylsulfoacétates, les alkylétherphosphates, les acylsarcosinates, les acyliséthionates et les N-acyltaurates). Dans un mode de mise en oeuvre, les groupements alkyle et acyle contiennent d'environ 8 à environ 24 atomes de carbone et les groupements aryle sont choisis parmi les groupements phényle et benzyle.

Parmi les tensioactifs anioniques pouvant également être utilisés, on peut également citer les sels d'acide gras (tels que les sels des acides oléïque, ricinoléïque, palmitique, stéarique, les acides d'huile de coprah et d'huile de coprah hydrogénée) ; et les acyl-lactylates dont le groupement acyle contient de 8 à 20 atomes de carbone. On peut également utiliser des tensioactifs faiblement anioniques, comme les acides alkyl-D-galactosiduroniques et leurs sels ainsi que les acides alkyl(C₆-C₂₄)éthercarboxyliques polyoxyalkylénés, les acides alkyl(C₆-C₂₄)aryléthercarboxyliques polyoxyalkylénés, les acides alkyl(C₆-C₂₄)amidoéthercarboxyliques polyoxyalkylénés et leurs sels, comme ceux contenant de 2 à 50 groupements oxyde d'éthylène, et leurs mélanges.

Dans un mode de mise en oeuvre, le(s) tensioactif(s) anionique(s) peut (peuvent) être choisi(s) parmi les sels d'alkylsulfate, les sels d'alkyléthersulfate, et leurs mélanges. Dans un autre mode de mise en oeuvre, le tensioactif anionique est choisi parmi les lauryléthersulfates de sodium, d'ammonium ou de magnésium.

### (ii) Tensioactif(s) non ionique(s) :

En ce qui concerne les tensioactifs non ioniques (voir « Handbook of Surfactants » par M.R. Porter, édité par Blackie & Son (Glasgow et Londres), 1991, pages 116 - 178), leur nature n'a pas de caractère critique. Ainsi, des exemples de tensioactifs non ioniques pouvant être utilisés seuls ou en mélange incluent les acides gras, les alkylphénols, les α-diols et les alcools ; qui ont tous une chaîne grasse contenant par exemple de 8 à 18 atomes de carbone et qui ont été polyéthoxylés, polypropoxylés, et/ou polyglycérolés, où le nombre de groupements d'oxyde d'éthylène ou d'oxyde de propylène varie de 2 à 50 et le nombre de groupements glycérol varie de 2 à 30. On peut également citer les copolymères d'oxyde d'éthylène et d'oxyde de propylène, les condensats d'oxyde d'éthylène et d'oxyde de propylène sur les alcools gras; les amides gras polyéthoxylés (tels que ceux ayant de 2 à 30 moles d'oxyde d'éthylène) ; les amides gras polyglycérolés contenant en moyenne 1 à 5, par exemple de 1,5 à 4 groupements glycérol ; les amines grasses polyéthoxylées (telles que celles ayant de 2 à 30 moles d'oxyde d'éthylène) ; les esters d'acide gras du sorbitane oxyéthylénés ayant de 2 à 30 moles d'oxyde d'éthylène; les esters d'acides gras du sucrose ; les esters d'acides gras de polyéthylèneglycol ; les alkylpolyglycosides ; les dérivés de N-alkylglucamine ; et les oxydes d'amines tels que les oxydes d'alkyl(C₁₀-C₁₄)amines et les oxydes de N-acylaminopropylmorpholine. Dans un mode de mise en oeuvre, les tensioactifs non ioniques sont choisis parmi les alkylpolyglycosides.

### (iii) Tensioactif(s) amphotère(s) :

Les tensioactifs amphotères, dont la nature n'a pas de caractère critique, peuvent être choisis par exemple parmi des dérivés d'amines secondaires ou tertiaires aliphatiques dans lesquels le radical aliphatique est choisi parmi des chaînes linéaires et ramifiées contenant 8 à 22 atomes de carbone et contenant au moins un groupement anionique hydrosoluble (par exemple des groupements carboxylate, sulfonate, sulfate, phosphate et phosphonate). Des exemples convenables incluent les alkyl(C₈-C₂₀)bétaïnes, les sulfobétaïnes, les alkyl(C₈-C₂₀)amidoalkyl(C₁-C₆)bétaïnes et les alkyl(C₈-C₂₀)amidoalkyl(C₁-C₆)sulfobétaïnes.

Parmi les dérivés d'amines, on peut citer les produits de formules (A) et (B) :

R₂-CONHCH₂CH₂-N⁺(R₃)(R₄)(CH₂COO⁻) (A)

dans laquelle R₂ est choisi parmi des groupements alkyle issus d'un acide R₂-COOH présent dans l'huile de coprah hydrolysée, des groupements heptyle, nonyle et undécyle, R₃ est choisi parmi des groupements β-hydroxyéthyle, et R₄ est choisi parmi des groupements carboxyméthyle ; et

R₅-CONHCH₂CH₂-N(B)(C) (B)

dans laquelle B est choisi parmi -CH₂CH₂OX', C est choisi parmi -(CH₂)_{z}-Y', avec z = 1 ou 2 ; X' est choisi parmi les groupements-CH₂CH₂-COOH et l'hydrogène ; Y' est choisi parmi les groupements -COOH et -CH₂-CHOH-SO₃H ; R₅ est choisi parmi les radicaux alkyle d'un acide R₉-COOH présent dans l'huile de coprah et dans l'huile de lin hydrolysée, les radicaux alkyle (tels que les radicaux alkyle en C₇, C₉, C₁₁ et C₁₃), et les radicaux alkyle en C₁₇ (tels que la forme iso et les radicaux C₁₇ insaturés).

Ces composés sont classés dans le dictionnaire CTFA, 5ème édition, 1993, sous les noms disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid, et cocoamphodipropionic acid. Un exemple utile peut être le cocoamphodiacétate vendu sous le nom commercial Miranol C2M concentré par la société Rhodia Chimie.

### (iv) Tensioactif(s) cationique(s)

Les tensioactifs cationiques peuvent être choisis parmi:
A) les sels d'ammonium quaternaires de formule (IX) ci-dessous : 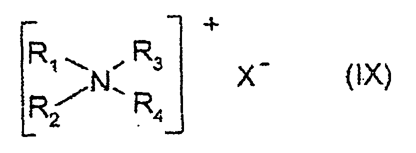 dans laquelle X⁻ est un anion choisi parmi des halogénures (tels que chlorure, bromure et iodure) et les alkyl(C₂-C₆)sulfates (tels que les méthylsulfates), les phosphates, les alkyl- et alkylarylsulfonates, les anions issus d'acides organiques (tels que l'acétate et le lactate), et soit:
   (i) R₁, R₂ et R₃ sont choisis indépendamment parmi des groupements aliphatiques linéaires et ramifiés contenant de 1 à 4 atomes de carbone et des groupements aromatiques (tels qu'aryles et alkylaryles). Les groupements aliphatiques peuvent comprendre des hétéroatomes tels que par exemple l'oxygène, l'azote et le soufre, et des halogènes. Les groupements aliphatiques peuvent être choisis par exemple parmi les groupements alkyle, alcoxy et alkylamide. R₄ peut être choisi parmi des groupements alkyle linéaires et ramifiés contenant de 12 à 30 atomes de carbone. Dans un mode de mise en oeuvre, le tensioactif cationique est choisi parmi les sels de béhényltriméthylammonium (par exemple chlorure) ; soit
   (ii) R₁ et R₂ sont choisis indépendamment parmi des groupements aliphatiques linéaires et ramifiés contenant de 1 à 4 atomes de carbone et des groupements aromatiques dont les aryles et les alkylaryles. Les groupements aliphatiques peuvent comprendre un ou plusieurs halogènes et hétéroatomes tels que par exemple l'oxygène, l'azote et le soufre. Les groupements aliphatiques peuvent être choisis par exemple parmi les groupements alkyle, alcoxy, alkylamide et hydroxyalkyle contenant d'environ 1 à 4 atomes de carbone. R₃ et R₄ sont choisis indépendamment parmi les groupements alkyle linéaires et ramifiés contenant de 12 à 30 atomes de carbone, lesdits groupements peuvent comprendre au moins une fonction provenant des fonctions esters et amides. Dans un mode de mise en oeuvre, R₃ et R₄ peuvent être choisis parmi les groupements alkyl(C₁₂-C₂₂)amidoalkyle(C₂-C₆) et alkyl(C₁₂-C₂₂)acétate.

   Dans un mode de mise en oeuvre, le tensioactif cationique est choisi parmi les sels de stéaramidopropyldiméthyl(myristylacétate)ammonium (par exemple chlorure). Dans un autre mode de mise en oeuvre, le tensioactif cationique est choisi parmi le chlorure de palmitamidopropyltrimonium, qui est vendu sous le nom Varisoft PTC par Degussa Goldschmidt;
B) les sels d'ammonium quaternaires de l'imidazolinium, comme, par exemple, celui de formule (X) : 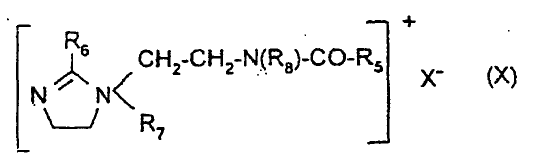 dans laquelle R₅ est choisi parmi les groupements alcényle et alkyle contenant de 8 à 30 atomes de carbone, tels que, par exemple, les dérivés d'acides gras du suif; R₆ est choisi parmi l'hydrogène, et les groupements alcényle et alkyle contenant de 8 à 30 atomes de carbone ; R₇ est choisi parmi les groupements alkyle en C₁-C₄ ; R₈ est choisi parmi l'hydrogène et les groupements alkyle en C₁-C₄ ; et X⁻ est un anion choisi parmi les halogénures, les phosphates, les acétates, les lactates, les alkylsulfates, les alkylsulfonates et les alkylarylsulfonates. Dans un mode de mise en oeuvre, R₅ et R₆ sont choisis indépendamment parmi les groupements alcényle et alkyle contenant de 12 à 21 atomes de carbone, tels que, par exemple, les dérivés d'acides gras du suif; R₇ est un groupement méthyle ; et R₈ est un hydrogène. Des exemples incluent Quatemium-27 (CTFA 1997) et Quaternium-83 (CTFA 1997), qui sont vendus sous les noms « Rewoquat » W75, W90, W75PG et W75HPG par la société Witco;
C) les sels de diammonium quaternaires de formule (XI) : 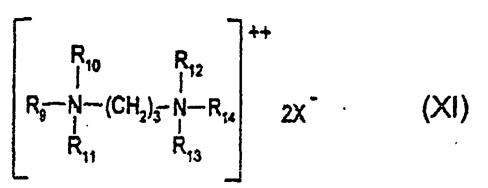 dans laquelle R₉ est choisi parmi les groupements aliphatiques contenant d'environ 16 à environ 30 atomes de carbone; R₁₀ R₁₁, R₁₂, R₁₃ et R₁₄ sont choisis indépendamment parmi l'hydrogène et les groupements alkyle contenant de 1 à 4 atomes de carbone ; et X⁻ est un anion choisi parmi les halogénures, les acétates, les phosphates, les nitrates et les méthylsulfates. De tels sels de diammonium quaternaires comprennent le dichlorure de propanesuifdiammonium ; et
D) les sels d'ammonium quaternaires contenant au moins une fonction ester de formule (XII) ci-dessous : 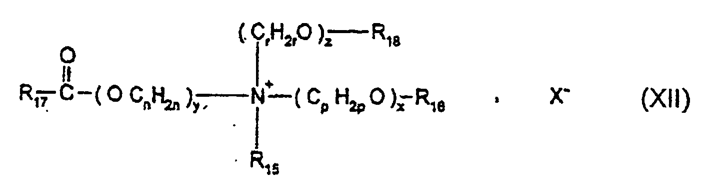 dans laquelle R₁₅ est choisi parmi les groupements alkyle en C₁-C₆, hydroxyalkyle en C₁-C₆ et dihydroxyalkyle ; R₁₆ est choisi parmi les groupements R₁₉-CO-, les groupements R₂₀ hydrocarbonés en C₁-C₂₂ linéaires et ramifiés, saturés et insaturés, et l'hydrogène ; R₁₈ est choisi parmi les groupements R₂₁-CO-, les groupements R₂₂ hydrocarbonés en C₁-C₆ linéaires et ramifiés, saturés et insaturés, et l'hydrogène ; R₁₇, R₁₉ et R₂₁ sont choisis indépendamment parmi les groupements hydrocarbonés en C₇-C₂₁, linéaires et ramifiés, saturés et insaturés ; n, p et r sont des entiers allant indépendamment de 2 à 6 ; y est un entier allant de 1 à 10 ; x et z sont des entiers allant indépendamment de 0 à 10; X⁻ est choisi parmi des anions simples et complexes, organiques et inorganiques; sous réserve que la somme x + y + z aille de 1 à 15, que lorsque x est 0, alors R₁₆ désigne R₂₀, et que lorsque z est 0, alors R₁₈ désigne R₂₂

Dans un mode de mise en oeuvre, R₁₅ est choisi parmi les groupements méthyle et éthyle ; x et y sont égaux à 1 ; z est égal à 0 ou 1 ; n, p et r sont égaux à 2; R₁₆ est choisi parmi les groupements R₁₉-CO-, méthyle, éthyle, et hydrocarbonés en C₁₄-C₂₂, et l'hydrogène ; R₁₇, R₁₉ et R₂₁ sont choisis indépendamment parmi les groupements hydrocarbonés en C₇-C₂₁ linéaires et ramifiés, saturés et insaturés ; R₁₈ est choisi parmi R₂₁-CO- et l'hydrogène.

Des exemples incluent des composés vendus sous les noms Dehyquart par la société Henkel, Stepanquat par la société Stepan, Noxamium par la société Ceca, et Rewoquat WE 18 par la société Rewo-Witco.

Dans un mode de mise en oeuvre, les tensioactifs cationiques sont choisis parmi le chlorure de béhényltriméthylammonium et le chlorure de stéaramidopropylméthyl(myristylacétate)ammonium vendus sous le nom « Ceraphyl 70 » par la société Van Dyk, et Quaternium-27 ou Quaternium-83 vendu par la société Witco.

Dans les compositions conformément à l'invention, on peut utiliser des mélanges de tensioactifs. Par exemple, des mélanges de tensioactifs anioniques et des mélanges de tensioactifs anioniques avec des tensioactifs amphotères et/ou non ioniques sont possibles.

Dans un mode de mise en oeuvre, on choisit un mélange comprenant au moins un tensioactif anionique et au moins un tensioactif amphotère. L'au moins un tensioactif anionique peut être choisi par exemple parmi les alkyl(C₁₂-C₁₄)sulfates de sodium, les alkyl(C₁₂-C₁₄)sulfates de triéthanolamine, les alkyl(C₁₂-C₁₄)sulfates d'ammonium, les alkyl(C₁₂-C₁₄)éthersulfates de sodium oxyéthylénés avec 2,2 moles d'oxyde d'éthylène, les alkyl(C₁₂-C₁₄)éthersulfates de triéthanolamine oxyéthylénés avec 2,2 moles d'oxyde d'éthylène, les alkyl(C₁₂-C₁₄)éthersulfates d'ammonium oxyéthylénés avec 2,2 moles d'oxyde d'éthylène, les cocoyliséthionates de sodium et les α-oléfines(C₁₄-C₁₆)sulfonates de sodium. L'au moins un tensioactif amphotère peut être choisi, par exemple, parmi les dérivés d'amines dénommés cocoamphodipropionates disodiques et cocoamphopropionates sodiques, tels que ceux vendus par la société Rhodia Chimie sous le nom commercial « Miranol C2M Conc. » sous forme de solution aqueuse à 38 % de matière active, ou sous le nom Miranol C32, et les tensioactifs amphotères du type zwittérionique, dont les alkylbétaïnes, tels que la cocobétaïne vendue sous le nom « Dehyton AB 30 » sous forme de solution aqueuse à 32 % de MA par la société Henkel ou la cocoamidopropylbétaïne. Dans un mode de mise en oeuvre, le mélange comprend la cocobétaïne et le lauryléthersulfate de sodium.
Selon un mode préféré de l'invention la composition selon l'invention comprend en outre au moins un agent conditionneur et au moins un agent tensioactif.

La composition selon l'invention peut être vaporisable, par exemple par une pompe, ou peut être une composition aérosol sous pression. Elle peut être vaporisable par une valve de distribution contrôlée par une tête de distribution, qui comprend à son tour une buse, qui vaporise la composition aérosol. Une composition vaporisable selon l'invention comprend un solvant approprié. De manière avantageuse, le solvant approprié comprend au moins un solvant choisi parmi l'eau et des alcools inférieurs. Conformément à l'invention, on entend par alcool inférieur un alcool aliphatique en C₁ à C₄, tel que l'éthanol.

Lorsque la composition vaporisable selon l'invention est une composition aérosol, elle comprend en outre une quantité appropriée d'agent propulseur. L'agent propulseur comprend des gaz comprimés ou liquéfiés, qui sont normalement utilisés pour la préparation de compositions aérosol. Les gaz convenables incluent l'air comprimé, le gaz carbonique, l'azote, et les gaz susceptibles d'être solubles dans la composition, tels que l'éther diméthylique, les hydrocarbures fluorés ou non fluorés, et leurs mélanges.

La présente invention fournit en outre un dispositif aérosol comprenant un récipient comprenant une composition aérosol, qui comprend une phase liquide (ou jus) comprenant au moins une matière de coiffage repositionnable, comme décrit ci-dessus, dans un milieu approprié et un agent propulseur, et un dispositif de distribution tel qu'une valve de distribution, pour la distribution de ladite composition aérosol à partir du récipient.

La présente invention fournit en outre un procédé de traitement de fibres kératiniques, notamment les cheveux, dans lequel la composition de coiffage repositionnable selon l'invention, telle que décrite ci-dessus, est appliquée sur les cheveux avant, pendant ou après la mise en forme de la coiffure.

La présente invention propose en outre l'utilisation d'une composition telle que décrite ci-dessus dans une formulation cosmétique de coiffage repositionnable ou pour sa préparation.

La détermination de la capacité d'une composition ayant un copolymère (méth)acrylique selon l'invention à donner un effet repositionnable peut être déterminée par un test *in vivo.*

Lorsque la composition est sous forme de lotion, par exemple, le test *in vivo* se déroule comme suit. Les cheveux du modèle sont lavés, puis divisés en deux portions symétriques, les côtés droit et gauche. La composition est appliquée sur un côté de la tête du modèle, puis rincée, alors qu'une composition de référence est appliquée sur l'autre côté de la tête. La composition de référence peut être choisie, par exemple, parmi l'eau, un produit commercial existant, ou une autre composition à l'étude. Le coiffeur sèche et coiffe les deux côtés de la tête. Les deux côtés de la tête sont évalués séparément pour l'effet de coiffage, les propriétés cosmétiques, et l'effet repositionnable. Par exemple, une fois séchés, les cheveux sont brossés dans différentes directions pour enlever la coiffure d'origine. Les cheveux sont alors brossés pour restaurer la coiffure d'origine. Le procédé pour enlever la coiffure, restaurer la coiffure, et évaluer le succès de restauration de la coiffure est répété au moins une fois de plus pour déterminer si la composition est une composition de coiffage repositionnable. Une composition de coiffage repositionnable permet (1) la restauration de la coiffure d'origine après brossage et (2) la création d'une nouvelle coiffure après brossage, qui peut également être restaurée après brossage. Si la composition à évaluer est sous une autre forme, telle qu'un shampoing ou un conditionneur, le test *in vivo* peut être modifié de façon appropriée par l'homme de l'art.

Il est entendu que l'hommè de l'art pourrait reconnaître que ce n'est pas toutes les formulations qui donneraient un effet repositionnable pour tous les types de cheveu pendant l'essai *in vivo* et saura comment formuler et évaluer des compositions de coiffage repositionnables en vue des différents paramètres capillaires, tels que la longueur (court contre long), le diamètre (fin contre épais), la structure (frisé contre défrisé), l'état (gras, sec ou normal) ; et si les cheveux sont colorés, décolorés, permanentés ou défrisés. Ainsi, l'essai *in vivo* peut nécessiter l'essai sur 10-20 individus différents.

### EXEMPLES :

Les compositions pour cheveux selon l'invention ont été produites avec différentes émulsions de (méth)acrylique.

### 1) Préparation d'émulsions d'acrylique :

### Exemple 1 :

Un mélange de 300 grammes d'acrylate de 2-éthylhexyle (2-EHA), 175 grammes d'acrylate de n-butyle (BA), et 25 grammes de méthacrylate de 2-hydroxyéthyle (HEMA) a été préparé, donnant 500 grammes d'une solution de monomère contenant 60/35/5 parties de 2-EHA/BA/HEMA. On a chargé 50 grammes de l'ensemble de la solution de monomère dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête a été placée sur la fiole et on a attaché un thermocouple, une entrée d'azote et un agitateur mécanique. Le contenu a été chauffé par des lampes à infrarouge jusqu'à environ 60°C tout en agitant à 350 t/min. On a chargé une solution de 1 gramme d'amorceur persulfate de potassium dans 20 grammes d'eau désionisée, on a fermé la fiole de manière étanche, et on a fait un vide dans la fiole quatre fois, en le cassant à chaque fois avec de l'azote. La fiole a été maintenue à 60°C pendant 20 minutes, puis chauffée à 80°C pendant 10 minutes pour donner un polymère amorce. On a préparé une pré-émulsion à partir des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion a été ajoutée goutte à goutte à la fiole à résine divisée de deux litres contenant le polymère amorce à un débit de 6 grammes par minute. L'addition a duré presque 2 heures. Après l'addition, la vitesse d'agitation a été réduite à 200 t/min et la réaction a été maintenue à 80°C pendant deux heures, puis le latex résultant a été filtré sur une toile de fromagerie doublée dans un bocal. On a noté des taux faibles de coagulum autour du thermocouple et de la pale d'agitation.

### Exemple 2 :

Un mélange de 300 grammes de (2-EHA), de 100 grammes d'acrylate d'isobutyle (IBA), de 75 grammes de (BA), et de 25 grammes de (HEMA) est préparé, donnant 500 grammes d'une solution de monomère contenant 60/20/15/5 parties de 2-EHA/IBA/BA/HEMA. A partir de l'ensemble de la solution de monomère, on charge 50 grammes dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête est placée sur la fiole et on attache un thermocouple, une entrée d'azote et un agitateur mécanique. Le contenu est chauffé par des lampes à infrarouge jusqu'à environ 60°C tout en agitant à 350 t/min. On charge une solution de 1 gramme d'amorceur persulfate de potassium dans 20 grammes d'eau désionisée, on ferme la fiole de manière étanche, et on fait un vide dans la fiole quatre fois, en le cassant à chaque fois avec de l'azote. On maintient la fiole à 60°C pendant 20 minutes, puis on chauffe à 80°C pendant 10 minutes pour donner un polymère amorce. On prépare une pré-émulsion des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion est ajoutée goutte à goutte à la fiole à résine divisée de deux litres contenant le polymère amorce à un débit de 6 grammes par minute. L'addition dure presque 2 heures. Après l'addition, la vitesse d'agitation est réduite à 200 t/min et la réaction doit être maintenue à 80°C pendant deux heures, puis le latex résultant sera filtré sur une toile de fromagerie doublée dans un bocal.

### Exemple 3 : (non invention)

Un mélange de 300 grammes de (IBA), de 150 grammes de (BA) et de 50 grammes d'acrylate de 2-hydroxyéthyle (HEA) est préparé, donnant 500 grammes d'une solution de monomère contenant 60/3/10 parties de IBA/BA/HEA. A partir de l'ensemble de la solution de monomère, on charge 50 grammes dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête est placée sur la fiole et on attache un thermocouple, une entrée d'azote et un agitateur mécanique. Le contenu est chauffé par des lampes à infrarouge jusqu'à environ 60°C tout en agitant à 350 t/min. On charge une solution de 1 gramme d'amorceur persulfate de potassium dans 20 grammes d'eau désionisée, on ferme la fiole de manière étanche, et on fait un vide dans la fiole quatre fois, en le cassant à chaque fois avec de l'azote. On maintient la fiole à 60°C pendant 20 minutes, puis on chauffe à 80°C pendant 10 minutes pour donner un polymère amorce. On prépare une pré-émulsion des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion est ajoutée goutte à goutte à la fiole à résine divisée de deux litres contenant le polymère amorce à un débit de 6 grammes par minute. L'addition dure presque 2 heures. Après l'addition, la vitesse d'agitation est réduite à 200 t/min et la réaction doit être maintenue à 80°C pendant deux heures, puis le latex résultant sera filtré sur une toile de fromagerie doublée dans un bocal.

### Exemple 4 :

Un mélange de 300 grammes d'acrylates de 2-carboxyéthyle (2-CEA), de 175 grammes de (BA), et de 25 grammes de (HEMA) est préparé, donnant 500 grammes d'une solution de monomère contenant 60/35/5 parties de 2-CEA/BA/HEMA. A partir de l'ensemble de la solution de monomère, on charge 50 grammes dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête est placée sur la fiole et on attache un thermocouple, une entrée d'azote et un agitateur mécanique. Le contenu est chauffé par des lampes à infrarouge jusqu'à environ 60°C tout en agitant à 350 t/min. On charge une solution de 1 gramme d'amorceur persulfate de potassium dans 20 grammes d'eau désionisée, on ferme la fiole de manière étanche, et on fait un vide dans la fiole quatre fois, en le cassant à chaque fois avec de l'azote. On maintient la fiole à 60°C pendant 20 minutes, puis on chauffe à 80°C pendant 10 minutes pour donner un polymère amorce. On prépare une pré-émulsion des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion est ajoutée goutte à goutte à la fiole à résine divisée de deux litres contenant le polymère amorce à un débit de 6 grammes par minute. L'addition dure presque 2 heures. Après l'addition, la vitesse d'agitation est réduite à 200 t/min et la réaction doit être maintenue à 80°C pendant deux heures, puis le latex résultant sera filtré sur une toile de fromagerie doublée dans un bocal.

### Exemple 5 :

Un mélange de 300 grammes de styrène (S), de 175 grammes (BA), et de 25 grammes de (HEMA) est préparé, donnant 500 grammes d'une solution de monomère contenant 60/35/5 parties de S/BA/HEMA. A partir de l'ensemble de la solution de monomère, on charge 50 grammes dans une fiole à résine divisée de deux litres ainsi que 380 grammes d'eau désionisée et 0,5 gramme de RHODACAL DS-10 (tensioactif dodécylbenzènesulfonate de sodium disponible dans le commerce auprès de Rhodia Chimie). La tête est placée sur la fiole et on attache un thermocouple, une entrée d'azote et un agitateur mécanique. Le contenu est chauffé par des lampes à infrarouge jusqu'à environ 60°C tout en agitant à 350 t/min. On charge une solution de 1 gramme d'amorceur persulfate de potassium dans 20 grammes d'eau désionisée, on ferme la fiole de manière étanche, et on fait un vide dans la fiole quatre fois, en le cassant à chaque fois avec de l'azote. On maintient la fiole à 60°C pendant 20 minutes, puis on chauffe à 80°C pendant 10 minutes pour donner un polymère amorce. On prépare une pré-émulsion des 450 grammes restants de la solution de monomère en la chargeant d'une solution de 4,5 grammes de dodécylbenzènesulfonate de sodium dans 211 grammes d'eau désionisée et en agitant sous l'azote. Cette pré-émulsion est ajoutée goutte à goutte à la fiole à résine divisée de deux litres contenant le polymère amorce à un débit de 6grammes par minute. L'addition dure presque 2 heures. Après l'addition, la vitesse d'agitation est réduite à 200 t/min et la réaction doit être maintenue à 80°C pendant deux heures, puis le latex résultant sera filtré sur une toile de fromagerie doublée dans un bocal.

### Exemple 6 :

On peut réaliser un mélange 50/50 de l'émulsion de l'exemple 1 et une dispersion comprenant AQ 1350 par Eastman Chemical Co. comme divulgué dans le document WO 98/38969.

### Exemple 7 :

On peut réaliser un mélange 25/75 de l'émulsion de l'exemple 1 et de l'émulsion de l'exemple 2.

### 2) Préparation des compositions de coiffage :

Cinq compositions de coiffage selon l'invention sous forme de shampoing ont été préparées en utilisant les composants et les quantités en % en poids énumérés ci-après. MA = matière active

| Formulation A: | |
|---|---|
| Polymère de l'Exemple 1 | 3,75 % de MA |
| MERQUAT 550 par NALCO | 0,28 % de MA |
| Lauryléthersulfate de sodium (2.2OE) | 9,0 % de MA |
| Cocobétaïne | 5,25 % de MA |
| VARISOFT PATC par Degussa Goldschmidt | 0,7 % de MA |
| Parfum, conservateur | qsp |
| Eau | qsp 100% |

| Formulation B : | |
|---|---|
| Polymère de l'Exemple 1 | 3,75 % de MA |
| MERQUAT 1050 par NALCO | 0,28 % de MA |
| Lauryléthersulfate de sodium (2.2OE) | 9,0 % de MA |
| Cocobétaïne | 5,25 % de MA |
| VARISOFT PATC par Degussa Goldschmidt | 0,7 % de MA |
| Parfum, conservateur | qsp |
| Eau | qsp 100% |

La formulation B donne un bon coiffage avec de bonnes propriétés cosmétiques et un effet repositionnable comparable à la formulation A.

D'autres formulations ont été fabriquées.

| Formulation C: | |
|---|---|
| Polymère de l'Exemple 1 | 2 % de MA |
| MERQUAT 550 par NALCO | 0,15 % de MA |
| Lauryléthersulfate de sodium (2.2OE) | 9,0 % de MA |
| Cocobétaïne | 5,25 % de MA |
| Parfum, conservateur | qsp |
| Eau | qsp 100 % |

La formulation C a donné un effet repositionnable.

| Formulation D : | |
|---|---|
| Polymère de l'Exemple 1 | 5 % de MA |
| MERQUAT 550 par NALCO | 0,28 % de MA |
| Lauryléthersulfate de sodium (2.2OE) | 9,0 % de MA |
| Cocobétaïne | 5,25 % de MA |
| VARISOFT PATC par Degussa Goldschmidt | 0,7 % de MA |
| Parfum, conservateur | qsp |
| Eau | qsp 100% |

| Formulation E: | |
|---|---|
| Polymère de l'Exemple 1 | 0,3 % de MA |
| MERQUAT 100 par NALCO | 0,1 % de MA |
| Lauryléthersulfate de sodium (2.2 OE) | 12,5 % de MA |
| Cocobétaïne | 2,5 % de MA |
| Eau | qsp 100 % |

La formulation E a donné un effet repositionnable.
On a préparé la formulation d'après-shampooing de composition suivante :

| Formulation F : | |
|---|---|
| Polymère de l'Exemple 1 | 0,3 % de MA |
| SALCARE SC 95 (CIBA) | 1,0 % de MA |
| Eau | qsp 100 % |

La formulation F a donné un effet déformable.

## Revendications

1. Composition de coiffage repositionnable rincée **caractérisée en ce qu'**elle comprend dans un véhicule cosmétiquement acceptable de 0,01 à 15% en poids par rapport au poids total de la composition d'au moins un copolymère (méth)acrylique, ledit copolymère (méth)acrylique comprend :
(a) de 10 à 50 % en poids de motifs issus d'au moins un monomère choisi parmi les monomères de (méth)acrylate de butyle,
(b) de 2 à 50 % en poids de motifs issus d'au moins un monomère choisi parmi les monomères de (méth)acrylate d'hydroxyalkyle, et
(c) de 30 à 80 % en poids de motifs issus d'au moins un monomère copolymérisable autres que lesdits monomères (a) et (b) choisi parmi les monomères (méth)acrylates d'isobornyle, les (méth)acrylates d'iso-octyle et les (méth)acrylates de 2-éthylhexyle, les monomères polaires choisis parmi les acides (méth)acryliques, les acides itaconiques, les N-vinylpyrrolidones, les N-vinylcaprolactames, les (méth)acrylamides, les (méth)acrylates de diméthylaminoéthyle, les acrylonitriles, les (méth)acrylates de 2-carboxyéthyle, les anhydrides maléiques, les monoacrylates de méthoxypolyéthyléneglycol 550, et les monomères polymérisables par voie radicalaire libre à insaturation éthylénique choisis parmi le styrène et les esters de vinyle en C₁-C_{4.}
à condition que les copolymères (méth)acryliques ne désignent pas les copolymères acrylate de n-butyle / acrylate de 2-hydroxyéthyle /méthacrylate de méthyle ayant un rapport de monomères en pourcentage en poids de 65/15/20 ou 75/15/10.

2. Composition selon la revendication 1, **caractérisée en ce que** ledit monomère cité dans (a) est choisi parmi les (méth)acrylates de n-butyle, les (méth)acrylates d'isobutyle, les (méth)acrylates de t-butyle et les (méth)acrylates de 2-méthylbutyle, de préférence l'acrylate de n-butyle.

3. Composition selon l'une quelconque des revendications 1 à 2, **caractérisée en ce que** ledit monomère cité dans (b) est choisi parmi les (méth)acrylates de 2-hydroxyéthyle et les (méth)acrylates d'hydroxypropyle, et de préférence le méthacrylate de 2-hydroxyéthyle.

4. Composition selon l'une quelconque des revendications 1 à 3, **caractérisée en ce que** lesdits monomères de (méth)acrylate de 2-éthylhexyl est l'acrylate de 2-éthythexyle.

5. Composition selon l'une quelconque des revendications 1 à 4, **caractérisée en ce que** lesdits monomères polaires sont choisis parmi les acides (méth)acryliques.

6. Composition selon l'une quelconque des revendications 1 à 5, **caractérisée en ce que** ledit copolymère (méth)acrylique est réticulé avec au moins un agent de réticulation polyfonctionnel.

7. Composition selon la revendication 6, **caractérisée en ce que** ledit agent de réticulation polyfonctionnel est choisi parmi le divinylbenzène, les diacrylates d'alkyle, les triacrylates d'alkyle, les tétra-acrylates d'alkyle, les cétones aromatiques à insaturation monoéthylénique, les aziridine-amides multifonctionnels et les agents de réticulation ions métalliques.

8. Composition selon l'une quelconque des revendication 1 à 7, **caractérisée en ce que** lesdits motifs issus dudit monomère cité dans (a) sont présents dans une quantité allant de 30 à 40 % en poids par rapport au poids total de monomères.

9. Composition selon l'une quelconque des revendications 1 à 8, **caractérisée en ce que** lesdits motifs issus dudit monomère cité dans (b) sont présents dans une quantité allant de 2 à 25 % en poids, de préférence de 2 à 10 % en poids par rapport au poids total de monomères.

10. Composition selon l'une quelconque des revendications 1 à 9, **caractérisée en ce que** lesdits motifs issus dudit monomère copolymérisable cité dans (c) sont présents dans une quantité allant de 50 à 70 % en poids par rapport au poids total de monomères.

11. Composition selon l'une quelconque des revendications 1 à 10, **caractérisée en ce que** ledit copolymère (méth)acrylique comprend :
(a) de 30 à 40 % en poids de motifs issus d'au moins un monomère choisi parmi les monomères d'acrylate de n-butyle.
(b) de 2 à 10 % en poids de motifs issus d'au moins un monomère choisi parmi les monomères de (méth)acrylale de 2-hydroxyéthyle, et
(c) de 50 à 70 % en poids de motifs issus d'au moins un monomère choisi parmi les monomères d'acrylate de 2-éthylhexyle.

12. Composition selon la revendication 1, **caractérisée en ce que** ledit au moins un copolymère (méth)acrylique a une Tg allant de -100°C à 15°C.

13. Composition selon l'une quelconque des revendications 1 à 12, **caractérisée en ce que** ledit copolymère (méth)acrylique est présent dans une quantité allant de 0,1 à 15 % en poids par rapport au poids total de la composition.

14. Composition selon l'une quelconque des revendications 1 à 13, comprenant en outre au moins un constituant choisi parmi les agents réducteurs ; les silanes : les matières grasses ; les épaississants ; les plastifiants ; les agents anti-mousse ; les agents hydratants ; les charges ; les filtres solaires ; les agents actifs de soin des cheveux ; les parfums ; les conservateurs : les tensioactifs cationiques, anioniques, non ioniques, et amphotères ; les polymères cationiques, anioniques, non ioniques, et amphotères ; les polyols ; les protéines ; les provitamines ; les vitamines ; les colorants : les produits de teinture ; les produits de décoloration ; et les agents de modification du pH.

15. Composition salon la revendication 14, **caractérisée en ce que** ledit constituant est choisi parmi les polymères cationiques.

16. Composition selon la revendication 15, **caractérisée en ce que** ledit polymère cationique est choisi parmi les dérivés d'éthers de cellulose quaternaires : la gomme de guar quaternisée ; les cyclopolymères cationiques, notamment les homopolyméres ou les copolymères du chlorure de diméthyldiallylammonium, les polymères quaternaires de vinylpyrrolidone et de vinylimidazole ; les polymères non réticulés et réticulés de sels de méthacryloyloxyalkyl(C₁-C₄)trialkyl(C₁-C₄)ammonium ; et des mélanges de ceux-ci.

17. Composition selon la revendication 16, **caractérisée en ce que** ledit polymère cationique est choisi parmi les homopolymères ou copolymères de chlorure de diméthyldiallylammonium.

18. Composition selon l'une quelconque des revendications 15 à 17, **caractérisée en ce que** ledit polymère cationique représente de 0,001 % à 20 % en poids, par exemple de 0,01 % à 10 % en poids, encore par exemple de 0,1 % à 3 % en poids, par rapport au poids total de la composition finale.

19. Composition selon l'une quelconque des revendications 1 à 18, comprenant en outre au moins un tensioactif choisi parmi les tensioactifs anioniques et cationiques.

20. Composition selon l'une quelconque des revendications 1 à 19, comprenant en outre au moins un agent conditionneur.

21. Composition selon l'une quelconque des revendications 1 à 20, comprenant au moins un agent conditionneur et au moins un tensioactif.

22. Composition selon l'une quelconque des revendications 14, 19 et 21, **caractérisée en ce que** ledit au moins un tensioactif est présent en une quantité allant de 0,1 % à 40 % en poids par rapport au poids total de la composition finale.

23. Composition selon l'une quelconque des revendications 1 à 22, **caractérisée en ce que** la composition est un shampoing.

24. Composition selon l'une quelconque des revendications 1 à 22, **caractérisée en ce que** la composition est un conditionneur.

25. Composition selon l'une quelconque des revendications 1 à 24, comprenant au moins un copolymère (méth)acrylique, **caractérisée en ce que** ladite composition est sous une forme choisie parmi les sprays, les aérosols, les mousses, les gels, les lotions, les crèmes, les dispersions et les émulsions.

26. Procédé de traitement cosmétique des cheveux, comprenant l'application sur les cheveux avant la mise en forme d'une coiffure desdits cheveux d'une composition selon les revendications 1 à 25, ladite composition étant rinçée.

27. Procédé de déformation des cheveux, comprenant :
(1) l'application sur les cheveux avant la mise en forme initiale de la coiffure d'une composition selon les revendications 1 à 25, la composition étant rinçée et
(2) ensuite la mise en forme de la coiffure au moins une fois, **caractérisé en ce qu'**on n'a pas besoin de composition ou de chaleur supplémentaire.

## Patentansprüche

1. Zusammensetzung für die Frisurengestaltung, die ausgespült wird, wobei die Frisur erneut gestaltet werden kann, **dadurch gekennzeichnet, dass** sie in einem kosmetisch akzeptablen Träger 0,01 bis 15 Gew.-% eines (Meth)acrylcopolymers, bezogen auf das Gesamtgewicht der Zusammensetzung, enthält, wobei das (Meth)acrylcopolymer umfasst:
(a) 10 bis 50 Gew.-% Einheiten, die von mindestens einem Monomer stammen, das unter den Butyl(meth)acrylat-Monomeren ausgewählt ist,
(b) 2 bis 50 Gew.-% Einheiten, die von mindestens einem Monomer stammen, das unter den Hydroxyalkyl(meth)acrylat-Monomeren ausgewählt ist, und
(c) 30 bis 80 Gew.% Einheiten, die von mindestens einem, von den Monomeren (a) und (b) verschiedenen, copolymerisierbaren Monomer stammen, das unter den folgenden Monomeren ausgewählt ist: Isobornyl(meth)acrylat, Isooctyl(meth)acrylat und 2-Ethylhexyl(meth)acrylat, polaren Monomeren, die unter (Meth)acrylsäure, Itaconsäure, N-Vinylpyrrolidon, N-Vinylcaprolactam, (Meth)acrylamiden, Dimethylaminoethyl(meth)acrylat, Acrylnitrilen, 2-Carboxyethyl(meth)acrylat, Maleinsäureanhydrid, dem Monoacrylat von Methoxypolyethylenglykol 550 ausgewählt sind, und auf radikalischem Wege polymerisierbaren Monomeren mit ethylenisch ungesättigter Bindung, die unter Styrol und den C₁₋₄-Vinylestern ausgewählt sind,
mit der Maßgabe, dass die (Meth)acrylcopolymere keine n-Butylacrylat/ 2-Hydroxyethylacrylat/Methylmethacrylat-Copolymere in einem prozentualen Verhältnis der Monomere von 65/15/20 oder 75/15/10 bedeuten.

2. Zusammensetzung nach Anspruch 1, **dadurch gekennzeichnet, dass** das unter (a) genannte Monomer unter n-Butyl(meth)acrylat, Isobutyl(meth)acrylat, t-Butyl(meth)acrylat und 2-Methylbutyl(meth)acrylat, vorzugsweise n-Butylacrylat ausgewählt ist.

3. Zusammensetzung nach einem der Ansprüche 1 bis 2, **dadurch gekennzeichnet, dass** das unter (b) genannte Monomer unter 2-Hydroxyethyl(meth)acrylat und Hydroxypropyl(meth)acrylat und vorzugsweise 2-Hydroxyethyl(meth)acrylat ausgewählt ist.

4. Zusammensetzung nach einem der Ansprüche 1 bis 3, **dadurch gekennzeichnet, dass** es sich bei den 2-Ethylhexyl(meth)acrylat-Monomeren um 2-Ethylhexylacrylat handelt.

5. Zusammensetzung nach einem der Ansprüche 1 bis 4, **dadurch gekennzeichnet, dass** die polaren Monomere unter (Meth)acrylsäure ausgewählt sind.

6. Zusammensetzung nach einem der Ansprüche 1 bis 5, **dadurch gekennzeichnet, dass** das (Meth)acrylcopolymer mit mindestens einem polyfunktionellen Vernetzungsmittel vernetzt ist.

7. Zusammensetzung nach Anspruch 6, **dadurch gekennzeichnet, dass** das polyfunktionelle Vernetzungsmittel unter Divinylbenzol, Alkyldiacrylaten, Alkyltriacrylaten, Alkyltetraacrylaten, aromatischen Ketonen mit monoethylenisch ungesättigter Bindung, multifunktionellen Aziridinamiden und Vernetzungsmitteln auf Metallionen-Basis ausgewählt ist.

8. Zusammensetzung nach einem der Ansprüche 1 bis 7, **dadurch gekennzeichnet, dass** die Einheiten, die von dem unter (a) genannten Monomer stammen, in einer Menge von 30 bis 40 Gew.-%, bezogen auf das Gesamtgewicht der Monomere, vorliegen.

9. Zusammensetzung nach einem der Ansprüche 1 bis 8, **dadurch gekennzeichnet, dass** die Einheiten, die von dem unter (b) genannten Monomer stammen, in einem Mengenanteil von 2 bis 25 Gew.-%, vorzugsweise 2 bis 10 Gew.-%, bezogen auf das Gesamtgewicht der Monomeren, vorliegen.

10. Zusammensetzung nach einem der Ansprüche 1 bis 9, **dadurch gekennzeichnet, dass** die Einheiten, die von dem unter (c) genannten copolymerisierbaren Monomer stammen, in einer Menge von 50 bis 70 Gew.-%, bezogen auf das Gesamtgewicht der Monomeren, enthalten sind.

11. Zusammensetzung nach einem der Ansprüche 1 bis 10, **dadurch gekennzeichnet, dass** das (Meth)acrylcopolymer umfasst:
(a) 30 bis 40 Gew.-% Einheiten, die von mindestens einem Monomer stammen, das unter den n-Butylacrylat-Monomeren ausgewählt ist,
(b) 2 bis 10 Gew.-% Einheiten, die von mindestens einem Monomer stammen, das unter den 2-Hydroxyethyl(meth)acrylat-Monomeren ausgewählt ist, und
(c) 50 bis 70 Gew.-% Einheiten, die von mindestens einem Monomer stammen, das unter den 2-Ethylhexylacrylat-Monomeren ausgewählt ist.

12. Zusammensetzung nach Anspruch 1, **dadurch gekennzeichnet, dass** das genannte, mindestens eine (Meth)acrylcopolymer eine Tg von mindestens -100 bis 15 °C aufweist.

13. Zusammensetzung nach einem der Ansprüche 1 bis 12, **dadurch gekennzeichnet, dass** das (Meth)acrylcopolymer in einer Menge enthalten ist, die im Bereich von 0,1 bis 15 Gew.-%, bezogen auf das Gesamtgewicht der Zusammensetzung, liegt.

14. Zusammensetzung nach einem der Ansprüche 1 bis 13, die ferner mindestens einen Bestandteil enthält, der unter den Reduktionsmitteln; Silanen; Fettsubstanzen; Verdickungsmitteln; Weichmachern, Schaumverhütungsmitteln; Hydratisierungsmitteln; Füllstoffen; Sonnenschutzfiltern; Wirkstoffen zur Pflege der Haare; Parfums; Konservierungsmitteln; kationischen, anionischen, nichtionischen und amphoteren grenzflächenaktiven Stoffen; kationischen, anionischen, nichtionischen, amphoteren Polymeren; Polyolen; Proteinen; Provitaminen; Vitaminen; Farbmitteln; Produkten zum Färben; Produkten zum Entfärben und Stoffen zur Einstellung des pH-Werts ausgewählt ist.

15. Zusammensetzung nach Anspruch 14, **dadurch gekennzeichnet, dass** der Bestandteil unter den kationischen Polymeren ausgewählt ist.

16. Zusammensetzung nach Anspruch 15, **dadurch gekennzeichnet, dass** das kationische Polymer unter den quartären Celluloseetherderivaten; quaternisiertem Guargummi; kationischen Cyclopolymeren, insbesondere Homopolymeren oder Copolymeren von Dimethyldiallylammoniumchlorid, quartären Polymeren von Vinylpyrrolidon und Vinylimidazol; nicht vernetzten und vernetzten Polymeren von Methacryloyloxyalkyl(C₁₋₄)trialkyl(C₁₋₄)ammoniumsalzen und Gemischen dieser Verbindungen ausgewählt ist.

17. Zusammensetzung nach Anspruch 16, **dadurch gekennzeichnet, dass** das kationische Polymer unter den Homopolymeren oder Copolymeren von Dimethyldiallylammoniumchlorid ausgewählt ist.

18. Zusammensetzung nach einem der Ansprüche 15 bis 17, **dadurch gekennzeichnet, dass** das kationische Polymer 0,001 bis 20 Gew.-%, beispielsweise 0,01 bis 10 Gew.-%, zum Beispiel 0,1 bis 3 Gew.-%, bezogen auf das Gesamtgewicht der fertigen Zusammensetzung, ausmacht.

19. Zusammensetzung nach einem der Ansprüche 1 bis 18, die ferner mindestens einen grenzflächenaktiven Stoff enthält, der unter den anionischen und kationischen grenzflächenaktiven Stoffen ausgewählt ist.

20. Zusammensetzung nach einem der Ansprüche 1 bis 19, die ferner mindestens ein Konditioniermittel enthält.

21. Zusammensetzung nach einem der Ansprüche 1 bis 20, die mindestens ein Konditioniermittel und mindestens einen grenzflächenaktiven Stoff enthält.

22. Zusammensetzung nach einem der Ansprüche 14, 19 und 21, **dadurch gekennzeichnet, dass** der mindestens eine grenzflächenaktive Stoff in einer Menge von 0,1 bis 40 Gew.-%, bezogen auf das Gesamtgewicht der fertigen Zusammensetzung, enthalten ist.

23. Zusammensetzung nach einem der Ansprüche 1 bis 22, **dadurch gekennzeichnet, dass** die Zusammensetzung ein Haarwaschmittel ist.

24. Zusammensetzung nach einem der Ansprüche 1 bis 22, **dadurch gekennzeichnet, dass** die Zusammensetzung ein Conditioner ist.

25. Zusammensetzung nach einem der Ansprüche 1 bis 24, die mindestens ein (Meth)acrylcopolymer enthält, **dadurch gekennzeichnet, dass** die Zusammensetzung in einer Form vorliegt, die unter den Sprays, Aerosolen, Schäumen, Gelen, Lotionen, Cremes, Dispersionen und Emulsionen ausgewählt ist.

26. Verfahren zur kosmetischen Behandlung der Haare, das das Auftragen einer Zusammensetzung nach einem der Ansprüche 1 bis 25 vor der Formgebung der Haare umfasst, wobei die Zusammensetzung ausgespült wird.

27. Verfahren zur Verformung der Haare, das umfasst:
(1) Aufbringen einer Zusammensetzung nach einem der Ansprüche 1 bis 25 auf die Haare, bevor die Frisur eine anfängliche Form erhalten hat, wobei die Zusammensetzung ausgespült wird, und
(2) Formgebung der Frisur mindestens einmal, **dadurch gekennzeichnet, dass** keine weitere Zusammensetzung oder zusätzlich Wärme erforderlich ist.

## Claims

1. Rinse-out repositionable styling composition, **characterized in that** it comprises, in a cosmetically suitable vehicle, from 0.01 to 15% by weight, with respect to the total weight of the composition, of at least one (meth)acrylic copolymer, the said (meth)acrylic copolymer comprising:
(a) from 10 to 50% by weight of units resulting from at least one monomer chosen from butyl (meth)acrylate monomers,
(b) from 2 to 50% by weight of units resulting from at least one monomer chosen from hydroxyalkyl (meth)acrylate monomers,
(c) from 30 to 80% by weight of units resulting from at least one copolymerizable monomer other than the said monomers (a) and (b) chosen from isobornyl (meth)acrylic monomers, isooctyl (meth)acrylates and 2-ethylhexyl (meth)acrylates, polar monomers chosen from (meth)acrylic acids, itaconic acids, N-vinylpyrrolidones, N-vinylcaprolactams, (meth)acrylamides, dimethylaminoethyl (meth)acrylates, acrylonitriles, 2-carboxyethyl (meth)acrylates, maleic anhydrides, methoxypolyethylene glycol 550 monoacrylates, and monomers which can be polymerized by the free radical route possessing ethylenic unsaturation which are chosen from styrene and C₁-C₄ vinyl esters,
provided that the (meth)acrylic copolymers do not denote n-butyl acrylate/2-hydroxyethyl acrylate/methyl methacrylate copolymers having a ratio of monomers as percentage by weight of 65/15/20 or 75/15/10.

2. Composition according to Claim 1, **characterized in that** the said monomer cited in (a) is chosen from n-butyl (meth)acrylates, isobutyl (meth)acrylates, t-butyl (meth)acrylates and 2-methylbutyl (meth)acrylates, preferably n-butyl acrylate.

3. Composition according to either one of Claims 1 and 2, **characterized in that** the said monomer cited in (b) is chosen from 2-hydroxyethyl (meth)acrylates and hydroxypropyl (meth)acrylates, preferably 2-hydroxyethyl methacrylate.

4. Composition according to any one of Claims 1 to 3, **characterized in that** the said 2-ethylhexyl (meth)acrylate monomers is 2-ethylhexyl acrylate.

5. Composition according to any one of Claims 1 to 4, **characterized in that** the said polar monomers are chosen from (meth)acrylic acids.

6. Composition according to any one of Claims 1 to 5, **characterized in that** the said (meth)acrylic copolymer is crosslinked with at least one polyfunctional crosslinking agent.

7. Composition according to Claim 6, **characterized in that** the said polyfunctional crosslinking agent is chosen from divinylbenzene, alkyl diacrylates, alkyl triacrylates, alkyl tetraacrylates, aromatic ketones possessing monoethylenic unsaturation, polyfunctional aziridine amides and metal ion crosslinking agents.

8. Composition according to any one of Claims 1 to 7, **characterized in that** the said units resulting from the said monomer cited in (a) are present in an amount ranging from 30 to 40% by weight, with respect to the total weight of monomers.

9. Composition according to any one of Claims 1 to 8, **characterized in that** the said units resulting from the said monomer cited in (b) are present in an amount ranging from 2 to 25% by weight, preferably from 2 to 10% by weight, with respect to the total weight of monomers.

10. Composition according to any one of Claims 1 to 9, **characterized in that** the said units resulting from the said copolymerizable monomer cited in (c) are present in an amount ranging from 50 to 70% by weight, with respect to the total weight of monomers.

11. Composition according to any one of Claims 1 to 10, **characterized in that** the said (meth)acrylic copolymer comprises:
(a) from 30 to 40% by weight of units resulting from at least one monomer chosen from n-butyl acrylate monomers,
(b) from 2 to 10% by weight of units resulting from at least one monomer chosen from 2-hydroxyethyl (meth)acrylate monomers,
(c) from 50 to 70% by weight of units resulting from at least one monomer chosen from 2-ethylhexyl acrylate monomers.

12. Composition according to Claim 1, **characterized in that** the said (meth)acrylic copolymer has a Tg ranging from -100°C to 15°C.

13. Composition according to any one of Claims 1 to 12, **characterized in that** the said (meth)acrylic copolymer is present in an amount ranging from 0.1 to 15% by weight, with respect to the total weight of the composition.

14. Composition according to any one of Claims 1 to 13, additionally comprising at least one constituent chosen from reducing agents, silanes, fatty substances, thickeners, plasticizers, antifoaming agents, moisturizing agents, fillers, sunscreens, agents which are active in caring for the hair, fragrances, preservatives, cationic, anionic, non-ionic and amphoteric surfactants, cationic, anionic, non-ionic and amphoteric polymers, polyols, proteins, provitamins, vitamins, colorants, dyeing products, bleaching products and pH-modifying agents.

15. Composition according to Claim 14, **characterized in that** the said constituent is chosen from cationic polymers.

16. Composition according to Claim 15, **characterized in that** the said cationic polymer is chosen from quaternary cellulose ether derivatives, quaternized guar gum, cationic cyclopolymers, in particular homopolymers or copolymers of dimethyldiallylammonium chloride, quaternary polymers of vinylpyrrolidone and of vinylimidazole, noncrosslinked and crosslinked polymers of methacryloyloxy (C₁-C₄)alkyltri(C₁-C₄)alkylammonium salts, and mixtures of these.

17. Composition according to Claim 16, **characterized in that** the said cationic polymer is chosen from homopolymers or copolymers of dimethyldiallylammonium chloride.

18. Composition according to any one of Claims 15 to 17, **characterized in that** the said cationic polymer represents from 0.001% to 20% by weight, for example from 0.01% to 10% by weight, for example again from 0.1% to 3% by weight, with respect to the total weight of the final composition.

19. Composition according to any one of Claims 1 to 18, additionally comprising at least one surfactant chosen from anionic and cationic surfactants.

20. Composition according to any one of Claims 1 to 19, additionally comprising at least one conditioning agent.

21. Composition according to any one of Claims 1 to 20, comprising at least one conditioning agent and at least one surfactant.

22. Composition according to any one of Claims 14, 19 and 21, **characterized in that** the said at least one surfactant is present in an amount ranging from 0.1% to 40% by weight, with respect to the total weight of the final composition.

23. Composition according to any one of Claims 1 to 22, **characterized in that** the composition is a shampoo.

24. Composition according to any one of Claims 1 to 22, **characterized in that** the composition is a conditioner.

25. Composition according to any one of Claims 1 to 24 comprising at least one (meth)acrylic copolymer, **characterized in that** the said composition is in a form chosen from sprays, aerosols, foams, gels, lotions, creams, dispersions and emulsions.

26. Method for the cosmetic treatment of the hair, comprising the application to the hair, before the shaping of a hairstyle of the said hair, of a composition according to Claims 1 to 25, the said composition being rinsed out.

27. Method for the deformation of the hair, comprising:
(1) the application to the hair, before the initial shaping of the hairstyle, of a composition according to Claims 1 to 25, the composition being rinsed out, and
(2) subsequently the shaping of the hairstyle at least once, **characterized in that** additional heat or composition is not required.
